# FASCICULE DE BREVET EUROPEEN

(11) **EP 0 518 722 B1**
(45) Date de publication et mention de la délivrance du brevet: **23.10.1996**
(21) Numéro de dépôt: 92401487.1
(22) Date de dépôt: 01.06.1992
(51) Int. Cl.: C07D 339/08, A61K 31/385, A61K 7/00

(54) **Rétinoides substitués par un cycle dithiane et leur utilisation, procédé de préparation desdits composés, compositions cosmétique et pharmaceutique les contenant et utilisation thérapeutique de cette dernière**
Dithian substituierte Retinoide, ihre Verwendung, Verfahren zu ihrer Herstellung, diese enthaltende kosmetische und pharmazeutische Zusammensetzungen und ihre therapeutische Verwendung
Dithiane substituted retinoides, their use, process of their preparation, cosmetic and pharmaceutical compositions containing them and their use in therapeutics

(30) Priorité: 14.06.1991 FR 9107292
(43) Date de publication de la demande: 16.12.1992
(73) Titulaire: L'OREAL, F-75008 Paris (FR)
(72) Inventeur: Solladie, Guy, F-67000 Strasbourg (FR); Berl, Valérie, F-68200 Mulhouse (FR); Maignan, Jean, F-93290 Tremblay-les-Gonesse (FR)
(74) Mandataire: Peuscet, Jacques

(56) Documents cités:
- EP-A- 0 292 350
- EP-A- 0 292 350
- EP-A- 0 391 033
- GB-A- 2 028 818
- CHEMICAL ABSTRACTS, vol. 92, no. 9, 3 Mars 1980, Columbus, Ohio, US; abstract no. 76738S, page 703 ;colonne 2 ;

## Description

La présente invention concerne des dérivés rétinoïdes substitués par un cycle dithiane, leur procédé de préparation, leur utilisation comme point de départ d'hydrolyse et leur utilisation en médecine humaine et vétérinaire et en cosmétique, sous forme de compositions.

La famille des rétinoïdes comprend des composés naturels, notamment la vitamine A ou rétinol, le rétinal et l'acide rétinoïque, et un certain nombre de dérivés obtenus par synthèse.

Les rétinoïdes sont, de façon connue, des composés utilisés pour le traitement cosmétique ou pharmaceutique des affections de la peau associées à des perturbations de la différenciation épidermique telles que le psoriasis ou l'acné. L'utilisation du rétinol et de ses dérivés, par exemple, est décrite dans EP-A-0 391 033. Il est très important de maîtriser la stéréochimie des différentes réactions de synthèse des rétinoïdes de façon à obtenir des rétinoïdes ayant une stéréospécificité déterminée et non des rétinoïdes sous forme d'un mélange de différents stéréoisomères. En effet, le mode d'utilisation des rétinoïdes peut ne pas être le même selon leur forme stéréochimique. Par exemple, cliniquement, l'acide rétinoïque tout-trans est utilisé sous forme topique et l'acide rétinoïque 13-cis est utilisé sous forme systémique.

La présente invention a pour objet des composés rétinoïques sulfurés, préparés sous une forme stéréochimique déterminée : tout-trans ou 13-cis, susceptibles par hydrolyse de se transformer en rétinal ou acide rétinoïque ayant la même forme stéréospécifique, et un procédé de fabrication permettant de préparer ces composés sous une forme stéréochimique déterminée à partir d'un composé de départ , dit "synthon", de forme stéréospécifique correspondante.

La présente invention a donc pour objet un composé rétinoïque stéréospécifique de formule : 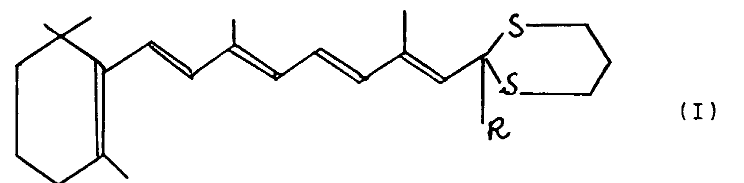 formule dans laquelle R est H ou un radical thioalkyle en C₁ - C₄.

Dans le présent texte et par convention, la position -trans du cycle dithiane est représentée en mettant l'axe de symétrie de ce cycle oblique vers le haut et la position -cis en orientant ledit axe vers le bas. Lorsque cet axe est représenté horizontal, la formule désigne indistinctement la forme stéréospécifique -cis ou la forme stéréospécifique -trans.

La présente invention concerne plus particulièrement le thioacétal du tout-trans rétinal de formule : 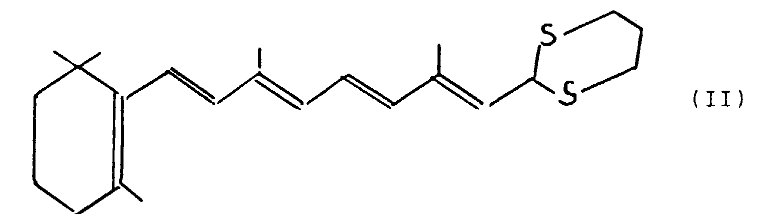 le thioacétal du 13-cis rétinal de formule : 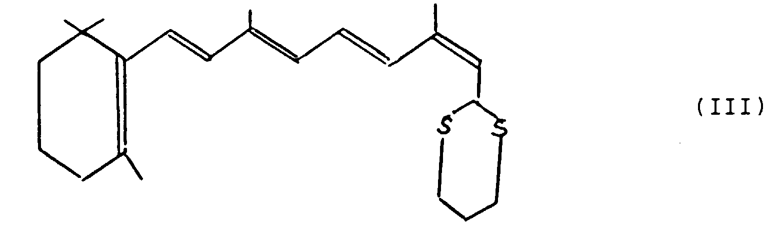 et l'orthothioester-trans de l'acide rétinoïque de formule :

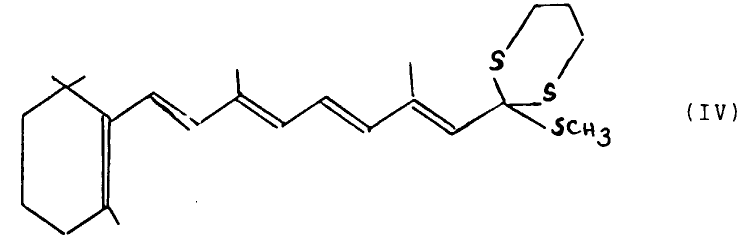

On a vérifié que ces produits ont une action rétinoïque comme leur structure permet de le supposer. D'autre part, par hydrolyse, les composés de formule (I) donnent le rétinal ou l'acide rétinoïque sous la forme stéréospécifique correspondant à celle du composé hydrolysé. Par exemple, les composés de formule (II) et (III) donnent respectivement le rétinal tout-trans et le rétinal 13-cis et le composé de formule (IV) de l'acide rétinoïque sous forme -trans.

L'invention a donc aussi pour objet l'utilisation des composés de formule (I) pour obtenir par hydrolyse un rétinal ou un acide rétinoïque stéréospécifique.

Les composés de formule (I) sont préparés à partir de diols ayant la stéréospécificité correspondante de formule : 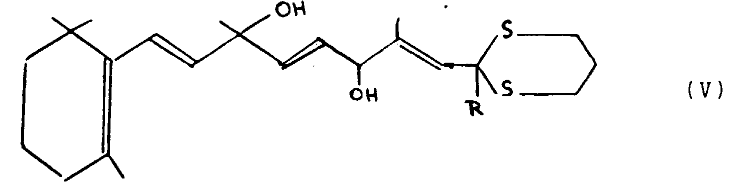 où R a la même signification que ci-dessus, par réduction à l'aide de titane "basse valence", de façon à éliminer les deux groupes hydroxyle et à obtenir une quatrième double liaison. Le titane "basse valence" est obtenu, de façon connue, par réduction de TiCl₃ ou TiCl₄ avec un métal tel que le magnésium, le potassium, le sodium, le lithium, le couple zinc--cuivre ou un hydrure de lithium et aluminium. On utilise, de préférence, un mélange de TiCl₃ et LiAlH₄ . Dans FR-A-2 615 509 est décrit un procédé de réduction d'un diol triénique en un composé rétinoïque comportant quatre doubles liaisons éthyléniques conjuguées. Selon la présente invention, on a trouvé que, dans le cas des diols de formule (V) ci-dessus, la réduction à l'aide de titane "basse valence" permettait de conserver la stéréospécificité. En d'autres termes, en utilisant par exemple un composé de formule (V) tout-trans, on obtient un rétinoïde tout-trans.

Les diols de formule (V) sont préparés par réaction d'un dithiane-propenal stéréospécifique de formule : 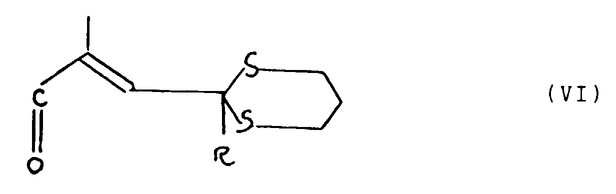 où R a la même signification que ci-dessus, avec l'éthynyl- β-ionol de formule : 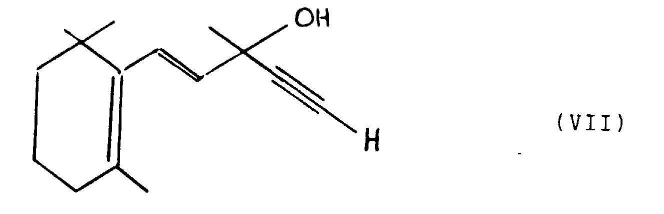 par réaction de Grignard en présence d'un organomagnésien, de façon à obtenir le composé acétylénique de formule : 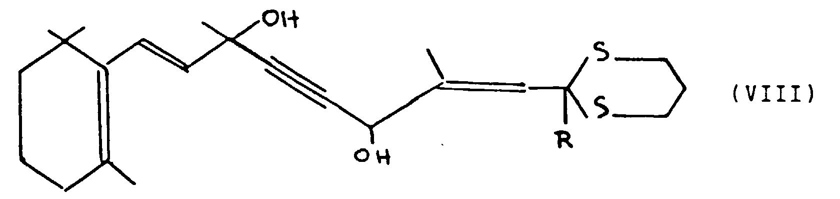 où R a la même signification que ci-dessus, puis par hydrogénation du composé de formule (VIII) pour obtenir le diol de formule (V). L'hydrogénation est, de préférence, effectuée à l'aide d'hydrure de lithium et d'aluminium.

Au cours de ces différentes réactions, la stéréospécificité du cycle dithiane par rapport à la double liaison la plus proche est respectée.

L'invention a donc également pour objet le procédé de préparation des composés de formule (I) à partir des composés de formule (VI) par les différentes étapes ci-dessus définies.

Certains des dithiane-propenals de formule (VI) utilisés sont connus. C'est le cas, notamment, du dithianepropenal-trans de formule : 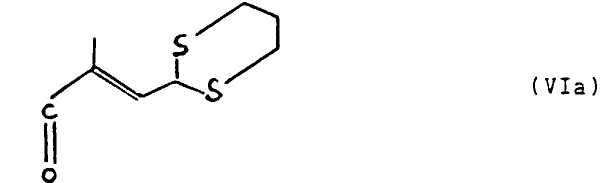 qui est décrit dans T.SAKO, K. HANAYAMA, T. FUJISAWA Tetrahedron Letters, 29, 2197 (1988) et K. RUSTEMEIER, E. BREITMAIER Chem. Ber, 115, 3898 (1982). D'autres dithiane-propenals sont des produits nouveaux, dont la synthèse est décrite en détail dans la demande de brevet (EP-A-0 518 724) qui est déposée le même jour que la présente demande par la demanderesse ; c'est, par exemple, le cas du dithianepropenal-cis de formule : 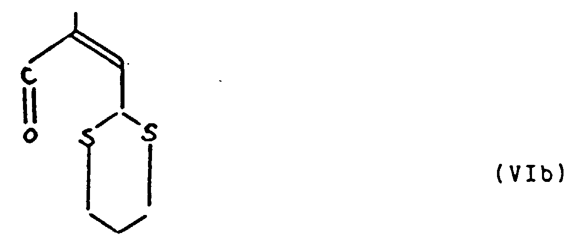 et de l'orthothioester-trans de formule :

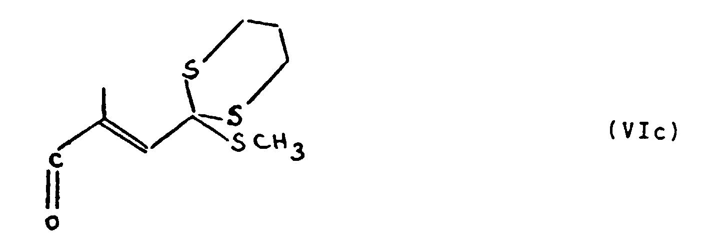

Les nouveaux composés de formule (I) trouvent aussi une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité anti-tumorale. En outre, ces composés peuvent être utilisés dans le traitement de l'atopie, qu'elle soit cutanée ou respiratoire , et du psoriasis rhumatoïde. Ils trouvent également une application dans le domaine ophtalmologique, notamment dans le traitement des cornéopathies.

Les composés selon l'invention conviennent particulièrement bien dans les domaines de traitement suivants :
1) pour traiter les affections dermatologiques liées à un désordre de la kératinisation portant sur la différenciation et sur la prolifération, notamment pour traiter les acnés vulgaires, comédoniennes, polymorphes, les acnés nodulokystiques, conglobata, les acnés séniles, les acnés secondaires telles que l'acné solaire, médicamenteuse ou professionnelle ;
2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen ;
3) pour traiter d'autres affections dermatologiques liées à un trouble de la kératinisation avec une composante inflammatoire et/ou immuno-allergique et, notamment, toutes les formes de psoriasis,qu'il soit cutané, muqueux ou unguéal, et même le rhumatisme psoriatique, ou encore l'atopie cutanée, telle que l'eczéma, ou l'atopie respiratoire ; mais aussi pour traiter certaines affections inflammatoires ne présentant pas de trouble de la kératinisation ;
4) pour traiter toutes les proliférations dermiques ou épidermiques,qu'elles soient bénignes , ou d'origine virale, telles que verrues vulgaires , les verrues planes et l'épidermodysplasie verruciforme, les proliférations pouvant également être induites par les ultra-violets notamment dans le cas des épithélioma baso et spino cellulaires ;
5) pour traiter d'autres désordres dermatologiques, tels que les dermatoses bulleuses et les maladies du collagène ;
6) pour traiter certains troubles ophtalmologiques, notamment les cornéopathies;
7) pour réparer et lutter contre le vieillissement de la peau, qu'il soit photoinduit ou chronologique ;
8) pour prévenir ou guérir les stigmates de l'atrophie épidermique et/ou dermique induite par les corticostéroïdes locaux ou systématiques, ou tout autre forme d'atrophie cutanée ;
9) pour favoriser la cicatrisation ;
10) pour lutter contre les troubles de la fonction sébacée,tels que la séborrhée de l'acné ou la séborrhée simple ;
11) pour traiter des situations cancéreuses ou précancéreuses,en particulier au niveau cutané ;
12) pour traiter des affections inflammatoires,telles que l'arthrite.

La présente invention a donc également pour objet une composition pharmaceutique contenant, dans un véhicule approprié pour une administration par voie entérale, parentérale, topique ou oculaire, au moins un composé de formule (I) telle que définie ci-dessus, ainsi que l'utilisation d'une telle composition pour le traitement des affections susmentionnées. Les composés de formule (I) sont généralement administrés à une dose journalière d'environ 0,01 mg à environ 100 mg/kg de poids corporel en 1 à 3 prises.

Pour une administration par voie entérale, les compositions pharmaceutiques de l'invention peuvent se présenter sous forme de comprimés, de gélules, de dragées, de sirops, de suspensions, de solutions, de poudres, de granulés, d'émulsions, de microsphères ou nanosphères ou de vésicules lipidiques ou polymériques permettant une libération contrôlée.

Pour une administration par voie parentérale, les compositions pharmaceutiques de l'invention peuvent se présenter sous forme de solutions ou suspensions pour perfusion ou pour injection.

Dans le cas d'une administration par voie topique, les compositions pharmaceutiques de l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions ; elles peuvent également se présenter sous forme de microsphères ou nanosphères ou de vésicules lipidiques ou polymériques ou de "patches" polymériques ou d'hydrogels permettant une libération contrôlée ; elles peuvent se présenter soit sous forme anhydre, soit sous forme aqueuse, selon l'indication clinique.

Pour une administration par voie oculaire, les compositions pharmaceutiques de l'invention sont principalement des collyres.

Les compositions pharmaceutiques de l'invention contiennent, à une concentration, de préférence,comprise entre 0,0001 et environ 5 % en poids par rapport au poids total de la composition, au moins un composé de formule (I) telle que définie ci-dessus.

Les composés de formule (I) trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention a donc enfin pour objet une composition cosmétique contenant, dans un véhicule cosmétique approprié, au moins un composé de formule (I), cette composition se présentant notamment sous forme d'une crème, d'un lait, d'une lotion, d'un gel, de microsphères ou nanosphères ou de vésicules lipidiques ou polymériques, d'un savon ou d'un shampooing. La concentration en composé(s) de formule (I) dans ces compositions cosmétiques est comprise, de préférence, entre 0,0001 et environ 3 % en poids par rapport au poids total de la composition.

Les compositions pharmaceutiques et cosmétiques selon l'invention peuvent contenir des additifs inertes ou des additifs pharmacodynamiquement ou cosmétiquement actifs.

Les exemples donnés ci-après, à titre purement illustratif et non limitatif, permettront de mieux comprendre l'invention.

### EXEMPLE 1 - Préparation du rétinoïde de formule (II)

### A) Préparation du synthon de départ constitué par le dithiane-propénal de formule :

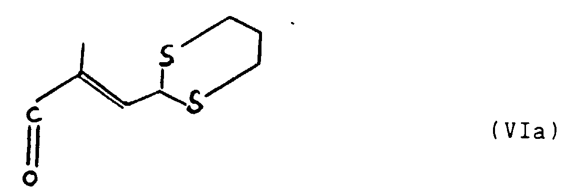

La préparation se fait selon le schéma donné ci-après.

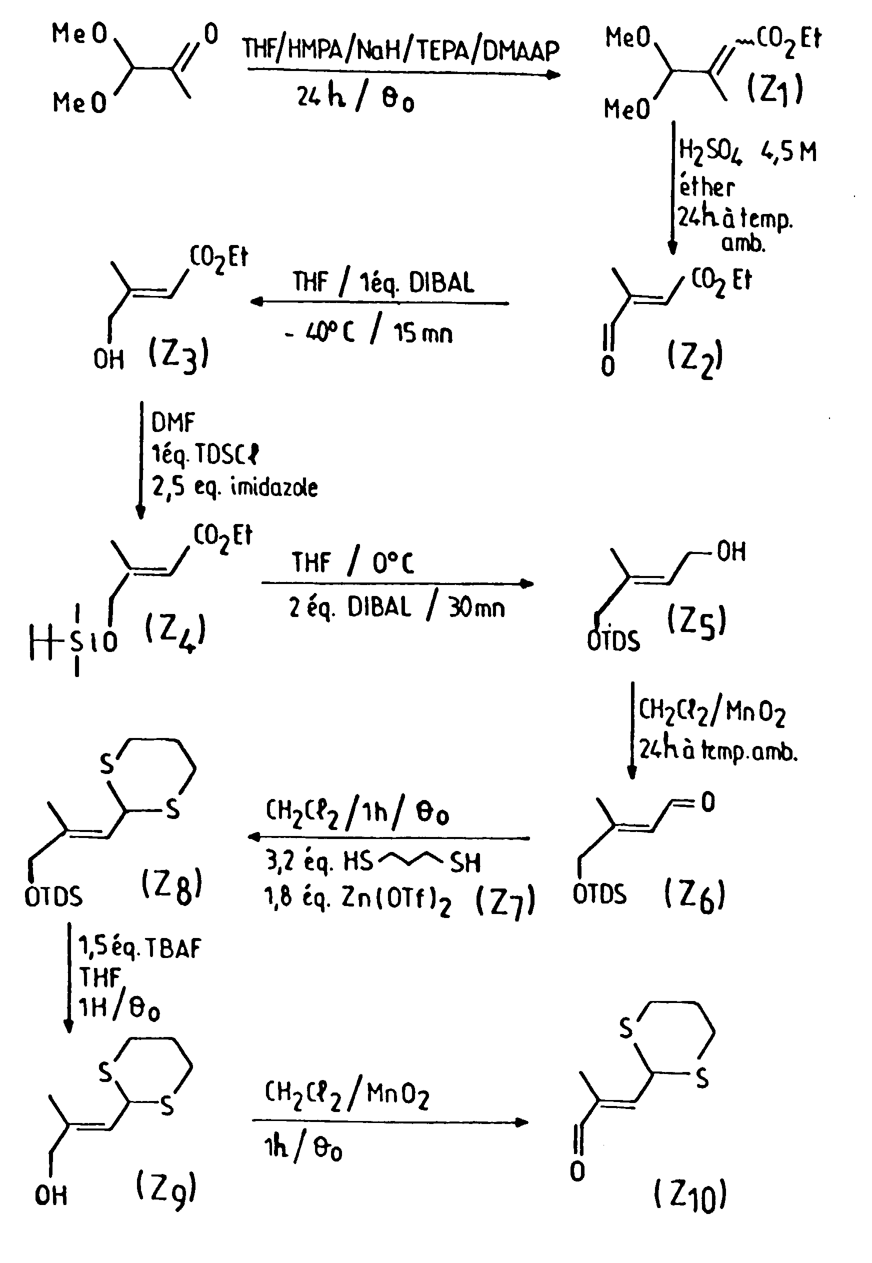

Dans ce schéma, les composés intermédiaires sont indiqués par les références (Zᵢ), i étant un nombre entier allant de 1 à 10 ; θₒ désigne la température ambiante. Le rendement total de la préparation est de 49 %.
1ère étape : Préparation du diméthyl-acétal du 4-oxo 3-méthyl(E/Z) 2-buténoate d'éthyle (Z₁) de formule : 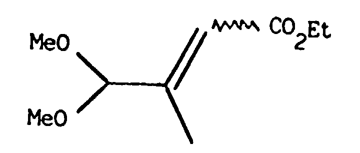 avec Et = éthyle
On met en suspension dans un ballon de l'hydrure de sodium (15,3 g - 0,64 mole) dans du tétrahydrofuranne (THF) anhydre (100 ml) sous argon. Après refroidissement du milieu à 0°C, un mélange d'hexaméthylphosphoramide (HMPA) (10 ml) et de triéthylphosphonoacétate (TEPA) (142 g - 0,64 mole) est ajouté au goutte à goutte sous agitation. Au bout d'une heure, la solution a pris une teinte brunâtre et un équivalent de diméthylacétal de l'aldéhyde pyruvique (DMAAP) (72,2 g - 0,64 mole) est ajouté lentement. L'agitation est maintenue pendant 24 heures à température ambiante. Au cours de la réaction, une gomme brune se dépose au fond du ballon réactionnel. Elle est dissoute par addition d'eau (150 ml). Le milieu est alors extrait à l'éther (2 x 100 ml). Les phases organiques rassemblées sont lavées avec une solution saturée en NaCl (2 x 150 ml), séchées sur Na₂SO₄ puis évaporées. On obtient 114 g d'un liquide légèrement jaune constitué d'un mélange des isomères E/Z dans le rapport 6/1.
Le rendement est de : 95 %
Les caractéristiques sont les suivantes :
a) Chromatographie en couche mince(CCM) sur gel de silice : (hexane/acétate d'éthyle : 70/30) Rf ≃ 0,66
b) M = 188,23 g
c) La structure a été confirmée par étude en résonance magnétique nucléaire RMN(¹H) et RMN(¹³C) (200 MHz) (CDCl₃) et étude du spectre infrarouge (IR) (CCl₄).

2ème étape :Préparation du γ-oxy sénécionate d'éthyle sous forme trans (Z₂)de formule : 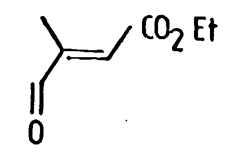 avec Et = éthyle
Un mélange 1/1 d'acide sulfurique 4,5 M et d'éther (400 ml) est refroidi à l'aide d'un bain de glace. L'acétal (Z₁) (mélange d'isomères E/Z) est ajouté au goutte à goutte sous agitation (20 g - 106 mmoles). Au bout de 24 heures, le milieu est extrait à l'éther (3 x 150 ml). Après lavage des phases organiques par une solution saturée en NaHCO₃(250 ml), par de l'eau (150 ml) et enfin par une solution saturée en NaCl (200 ml), puis séchage sur sulfate de sodium, le solvant est évaporé. On obtient 11,75 g d'un liquide jaune très pale.
Le rendement est de : 78 %
Les caractéristiques du produit sont les suivantes :
a) Chromatographie en couche mince (hexane/acétate d'éthyle : 70/30) Rf ≃ 0,66
b) Masse moléculaire M = 142,16 g
c) La structure a été confirmée par étude en RMN (¹H) (200 MHz) (CDCl₃) et étude du spectre infrarouge (CCl₄).

3ème étape :Préparation du γ-ol sénécionate d'éthyle trans (Z₃) de formule : 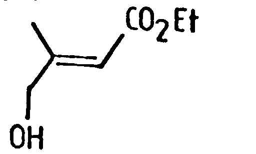 avec Et = éthyle
L'aldéhyde (Z₂) (10 g - 0,07 mole) est mis en solution dans du tétrahydrofuranne (THF) anhydre (300 ml) sous argon. Après refroidissement du milieu à -40°C, un équivalent d'hydrure de diisobutylaluminium (DIBAL) (70,3 ml de solution 1M dans le toluène) est additionné au goutte à goutte rapide. Après 15 minutes à cette température, le milieu est hydrolysé par addition lente de méthanol (20 ml), puis d'eau (100 ml). Dès que la température a atteint 0°C, 100 ml d'éther sont ajoutés puis, sous vigoureuse agitation, de l'acide chlorhydrique à 10 % en volume jusqu'à obtention de deux phases limpides. La phase aqueuse est extraite à l'éther (3 x 100 ml). Les phases organiques rassemblées sont lavées par une solution saturée en NaCl (150 ml), séchées sur Na₂SO₄, puis concentrées. Le produit brut réactionnel est chromatographié sur colonne de silice (éluant = hexane/acétate d'éthyle : 70/30). On obtient 8,75 g d'un liquide jaune.
Le rendement est de : 86 %
Les caractéristiques du produit sont les suivantes :
a) chromatographie en couche mince(hexane/acétate d'éthyle : 70/30) Rf ≃ 0,27
b) M = 144,17 g
c) La structure a été confirmée par RMN(¹H et ¹³C) (200 MHz) (CDCl₃) et spectre infrarouge (IR).
d) L'analyse élémentaire donne les résultats suivants :

| % | Calculé | Trouvé |
|---|---|---|
| C | 58,32 | 58,60 |
| H | 8,39 | 8,63 |

4ème étape : Préparation du t-hexyl diméthyl silylol sénécionate d'éthyle trans (Z₄) de formule : 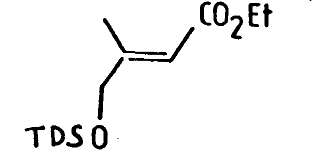 avec TDS = t-hexyl diméthyl silyl et Et = éthyle
A l'alcool (Z₃) (8,7 g - 0,06 mole) en solution dans du diméthylformamide (DMF) distillé sur tamis moléculaire (160 ml) et sous argon est ajouté du MgSO₄ flambé sous vide (environ 6 g). Le milieu est agité pendant 10 minutes à température ambiante, puis un équivalent de chlorure de t-hexyldiméthylsilyle (11,85 ml) suivi de 2,5 équivalents d'imidazole (10,27 g) sont additionnés. Après 0,5 h d'agitation à température ambiante, le mélange réactionnel est dilué par de l'eau (100 ml) et extrait à l'éther (4 x 100 ml). Les phases organiques rassemblées sont lavées par une solution saturée en NH₄Cl (2 x 100 ml), une solution saturée en NaCl (100 ml), séchées sur Na₂SO₄, puis concentrées. On obtient 15,45 g d'un liquide jaune pale , qui peut être purifié par chromatographie sur colonne de silice (éluant = hexane/éther : 95/5).
Le rendement est de : 90 %
Les caractéristiques du produit sont les suivantes :
a) CMM (hexane/acétate d'éthyle : 70/30)
Rf ≃ 0,85
b) M = 286,49 g
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 62,89 | 62,90 |
| H | 10,55 | 10,69 |

5ème étape : Préparation de t-hexyl diméthyl silylol sénécionol trans (Z₅) de formule : 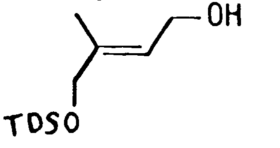 avec TDS = t-hexyl diméthyl silyl
L'ester (Z₄) (10 g - 0,035 mole) est mis en solution dans du tétrahydrofuranne (THF) anhydre (200 ml) sous argon. Après refroidissement du milieu à 0°C, 2,2 équivalents d'hydrure de diisobutylaluminium (DIBAL) (77 ml de solution 1M dans le toluène) sont additionnés au goutte à goutte rapide. Après 0,5 h la réaction est complète et le milieu est hydrolysé par addition lente de méthanol (3,7 ml). Le tout est versé sur un mélange d'acétate d'éthyle (1500 ml) et d'une solution saturée en tartrate de sodium (190 ml). Une agitation vigoureuse de ce milieu durant 1 h permet de "casser" le gel qui s'est formé. La phase aqueuse est alors extraite à l'acétate d'éthyle (3 x 150 ml). Les phases organiques rassemblées sont lavées avec une solution saturée en NaVl (150 ml), séchées sur Na₂ SO₄, puis concentrées. On obtient 8,55 g de produit (Z₅).
Les caractéristiques du produit obtenu sont les suivantes :
a) CCM (hexane/acétate d'éthyle : 70/30)
Rf ≃ 0,51
b) M = 244,46 g
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 63,88 | 64,01 |
| H | 11,54 | 11,75 |

6ème étape : Préparation du t-hexyl diméthyl silylol sénécional trans (Z₆) de formule : 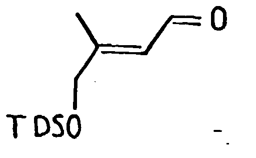 avec TDS = t-hexyl diméthyl silyl
L'alcool (Z₅) (7 g - 0,0286 mole) est dissous dans du CH₂Cl₂ (200 ml) sous argon. Environ 5 équivalents de dioxyde de manganèse (12 g) sont ajoutés au milieu sous vigoureuse agitation. Après 24 h à température ambiante, la suspension est filtrée sur une petite couche de silice. La silice est abondamment lavée à l'acétate d'éthyle. Le filtrat est concentré sous vide en évitant de chauffer. On récupère 6,94 g d'un liquide jaune légèrement volatil. Les caractéristiques du produit obtenu sont les suivantes :
a) CCM (hexane/acétate d'éthyle : 70/30)
Rf ≃ 0,73
b) M = 242,44 g
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 64,40 | 64,11 |
| H | 10,81 | 10,97 |

7ème étape : Préparation du triflate de zinc (Z₇): Zn(OTf)₂.
Un équivalent de carbonate de zinc (17,4 g - 0,139 mole) est mis en solution dans du méthanol sec (250 ml). A l'aide d'une ampoule à brome, 1,4 équivalent d'acide triflique (50 g - 0,195 mole) y sont ajoutés au goutte à goutte lent. La réaction est exothermique et un fort dégagement de CO₂ est observé. Le milieu est agité pendant 20 minutes à température ambiante, puis 2 h à reflux. Le méthanol est alors évaporé. Le résidu est séché par chauffage 2,5 h à 130°C sous 330 Pascals. On obtient 50,4 g d'une poudre blanche (M = 363,53 g).
8ème étape : Préparation du (E) 3-(1,3-dithiane 2-yl) 2-méthyl du t-hexyl diméthyl silylpropenol (Z₈) de formule : 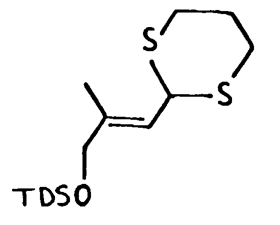 avec TDS = t-hexyl diméthyl silyl
3,2 équivalents de 1,3-propane dithiol (8,9 ml - 0,088 mole) et 1,2 équivalent de Zn(OTf)₂ (12,05 g - 0,033 mole) sont mis en solution dans du CH₂Cl₂ distillé (200 ml) sous argon. Après agitation du milieu pendant 15 minutes à température ambiante, un équivalent de l'aldéhyde (Z₆) (6,7 g - 0,0276 mole) en solution dans du CH₂Cl₂ (20 ml) est additionné au goutte à goutte rapide. La réaction est complète après 0,5 h et le milieu est dilué par addition d'eau (150 ml). La phase aqueuse est extraite par un mélange d'hexane/éther 1/1 (3 x 150 ml). Les phases organiques rassemblées sont lavées par de l'acide chlorhydrique à 10 % (150 ml) afin de "casser" l'émulsion qui s'est formée, puis par de la soude 0,5 M (3 x 200 ml), une solution saturée en NaHCO₃ (150 ml), de l'eau (2 x 150 ml) et enfin une solution saturée en NaCl (150 ml). Après séchage sur Na₂SO₄ et évaporation du solvant, 8,73 g d'une huile incolore est isolée.
Le rendement est de : 95 %
Les caractéristiques du produit obtenu sont les suivantes :
a) CCM (hexane/acétate d'éthyle : 90/10)
Rf ≃ 0,58
b) M = 332,65 g .
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 57,77 | 59,79 |
| H | 9,7 | 9,56 |

9ème étape : Préparation du (E) 3-(1,3-dithiane 2-yl) 2-méthylpropenol (Z₉) de formule : 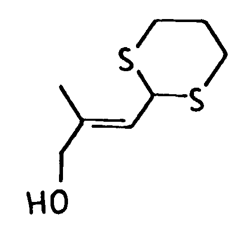 A un équivalent du composé silylé (Z₈) (8,5 g - 0,0255 mole), dissous dans du tétrahydrofuranne (THF) anhydre (80 ml) et sous argon, on ajoute 1,5 équivalent de fluorure de tétrabutyle ammonium (38,3 ml de solution 1M dans du THF). Le milieu réactionnel est agité pendant 1 h à température ambiante puis passé au travers d'une petite couche de célite. Le ballon réactionnel et la célite sont rincés à l'éther. Après concentration du filtrat, on récupère 4,86g d'un liquide jaune pâle.
Les caractéristiques du produit obtenu sont les suivantes :
a) CCM (hexane/acétate d'éthyle : 70/30)
Rf ≃ 0,29
b) M = 190,33 g
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 50,49 | 50,52 |
| H | 7,41 | 7,57 |

10ème étape - Préparation du (E) 3-(1,3-dithiane 2-yl) 2-méthylpropenal (Z₁₀) de formule : 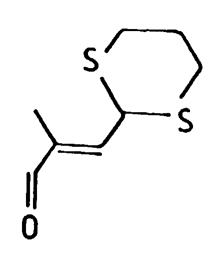
L'alcool (Z₉) (4,8 g - 0,025 mole) est dissous dans du CH₂ Cl₂ distillé (150 ml) sous argon. Environ cinq équivalents de dioxyde de manganèse (11 g) sont ajoutés au milieu sous vigoureuse agitation. Après 4 h à température ambiante, la suspension est filtrée sur une petite couche de silice. La silice est abondamment lavée à l'acétate d'éthyle. Le filtrat est concentré sous vide. On obtient 4,24 g de cristaux beiges.
Le rendement est de : 90 %
Les caractéristiques du produit obtenu sont les suivantes :
a) CCM (hexane/acétate d'éthyle : 70/30)
Rf ≃ 0,52
b) Point de fusion Pf = 38-39°C
c) M = 188,32 g
d) La structure a été confirmée par RMN(¹H et¹³C) (200 MHz) (CDCl₃) et par IR.
e) L'analyse élémentaire donne les résultats suivants :

| % | Calculé | Trouvé |
|---|---|---|
| C | 51,02 | 51,20 |
| H | 6,42 | 6,47 |

### B) Préparation du rétinoide de formule (II) :

La préparation est effectuée selon le schéma suivant :

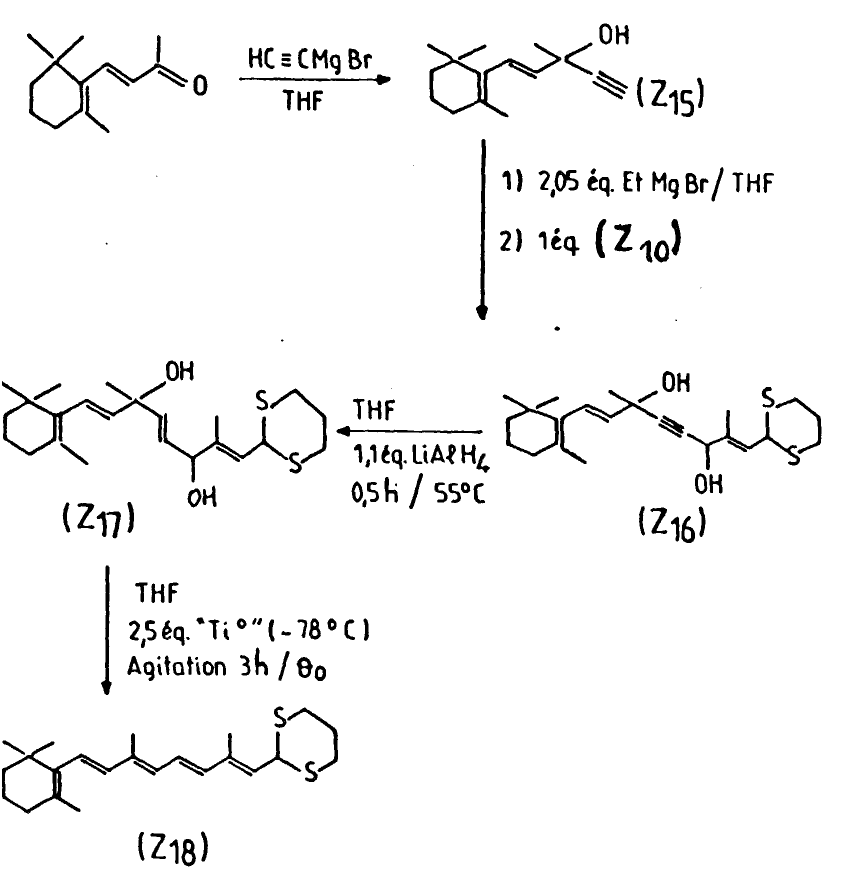

Dans ce schéma, les composés intermédiaires sont indiqués par les références (Zᵢ), i étant un nombre entier allant de 15 à 18 ; θₒ désigne la température ambiante.
1ère étape : Préparation de l'éthynyl-β-ionol(Z₁₅) de formule : 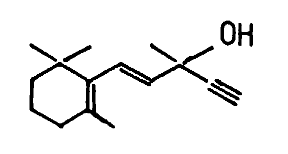
Du magnésium sec (5,1 g - 0,210 mole) et du tétrahydrofuranne (THF) anhydre (100 ml) sont placés dans un tricol de 250 cm³ , équipé d'une ampoule à brome et d'un réfrigérant. Une solution de bromure d'éthyle (15,7 ml - 0,210 mole) dans du THF anhydre (30 ml) est alors lentement ajoutée sous argon. Après réaction du magnésium, le milieu est maintenu pendant 1 heure à température ambiante.
D'autre part, un tricol de 500 cm³ est équipé d'un réfrigérant relié à un bulleur à huile, d'une ampoule à brome et d'un diffuseur à gaz pour acétylène (l'acétylène passant au préalable dans un piège à -78°C). L'acétylène est alors dissous pendant 1 h dans du THF anhydre (200 ml) entre -30 et -20°C. Après avoir enlevé le bain réfrigérant, on ajoute le magnésien au goutte à goutte rapide, toujours sous courant d'acétylène. La température est maintenue au-dessous de 35°C.
Après addition du magnésien, le milieu est agité pendant 30 minutes à température ambiante. Puis, la β-ionone (21,2 ml - 0,104 mole) en solution dans du THF anhydre (20 ml) est ajoutée au goutte à goutte pendant 30 minutes. Toujours sous courant d'acétylène, la réaction est terminée au bout d'une heure et la solution jaune-verte obtenue est versée lentement sur une solution saturée en NH₄Cl (250 ml). Le milieu est extrait à l'éther (3 x 50 ml), lavé avec de l'eau (50 ml), puis avec une solution saturée en NaCl (100 ml), séché sur Na₂SO₄ et enfin concentré. Le résidu est distillé (125°C/395 Pascals) afin d'obtenir une huile incolore. On obtient 18,16 g de produit. Le composé est conservé au congélateur et à l'abri de la lumière.
Le rendement est de : 80 %
Les caractéristiques du produit obtenu sont les suivantes :
a) CCM (hexane/acétate d'éthyle : 70/30)
   Rf ≃ 0,64
b) Pf = 21°C
c) M = 218,34 g
d ) La structure a été confirmée par RMN(¹H et¹³C) (200 MHz) (CDCl₃ ) et par IR.

2ème étape : Préparation du composé (Z₁₆)de formule : 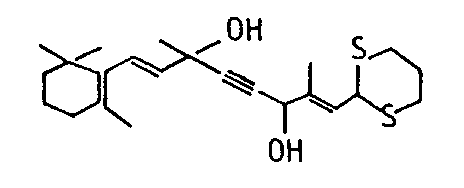
Dans un premier temps, un réactif de Grignard est préparé à partir de 2,05 équivalents de magnésium (1,13 g - 0,047 mole) dans du tétrahydrofuranne (THF) anhydre (20 ml) et sous argon. 2,1 équivalents de bromoéthane en solution dans du THF anhydre (20 ml) sont ajoutés au goutte à goutte. Dès que tout le magnésium est dissous, 1 équivalent du composé (Z₁₅) (5 g - 0,0229 mole) en solution dans du THF (40 ml) est additionné lentement. Un dégagement d'éthane est observé. Après 1,5 h de réaction à température ambiante, 1 équivalent d'aldéhyde (Z₁₀) (4,31 g-0,0229 mole) en solution dans du THF (40 ml) est ajouté au milieu rapidement. Après 1 h, la teneur en composé (Z₁₆) n'évoluant plus comme constaté par chromatographie en couche mince, le milieu est hydrolysé par addition lente d'une solution saturée en NH₄ Cl (40 ml), puis d'eau (40 ml).
La phase aqueuse est extraite à l'éther (3 x 80 ml). Les phases organiques rassemblées sont lavées avec une solution saturée en NaCl (100 ml), séchées sur Na₂SO₄, puis concentrées. Le résidu est purifié par flash-chromatographie (éluant = hexane/acétate d'éthyle : 85/15). On obtient 6,80 g d'une huile jaune très "mousseuse". Elle peut être conservée plusieurs mois au congélateur, mais elle se dégrade très vite à température ambiante.
Les caractéristiques du produit obtenu sont les suivantes :
a) CCM (hexane/acétate d'éthyle : 70/30)
   Rf ≃ 0,24
b) M = 406,66g
c) La structure a été confirmée par RMN(¹H) (200 MHz) (CDCl₃) et par IR.

3éme étape : Préparation du composé (Z₁₇) de formule : 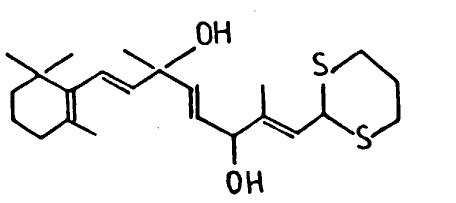
A 1 équivalent du composé (Z₁₆) (6,5 g - 0,016 mole) en solution composé (Z₁₆) (6,5 g - 0,016 mole) en solution dans du tétrahydrofuranne anhydre (800 ml) sous argon, on ajoute au goutte à goutte 1,1 équivalent de LiAlH₄ ajoute au de solution goutte dans équivalent de LiAlH₄ (13,6 ml de solution 1,29M dans l'éther). L'addition terminée, le ballon équipé d'un réfrigérant est plongé dans un bain préalablement chauffé à 55°C. A cette température, le milieu jaune prend peu à peu une couleur violette. Après 0,5h, la réaction est totale et le milieu est prudemment hydrolysé par addition d'une solution saturée en NH₄Cl (150 ml), suivie d'eau (50 ml). La phase aqueuse est extraite à l'éther (3 x 100 ml). Les phases organiques rassemblées sont lavées par une solution saturée en NaCl (100 ml), séchées sur Na₂SO₄, puis concentrées. Le résidu est purifié par flash-chromatographie (éluant = hexane/acétate d'éthyle : 90/10). On obtient 4,6 g d'une huile jaune. Elle peut être conservée plusieurs mois au congélateur, mais elle se dégrade très vite à température ambiante.
Le rendement est de : 70 %
Les caractéristiques du produit obtenu sont les suivantes :
a) CCM (hexane/acétate d'éthyle : 70/30)
   Rf ≃ 0,20
b) M = 408,67 g
c) La structure a été confirmée par RMN(¹H) (200 MHz) (CDCl₃) et par IR.

4ème étape : Obtention du rétinoide (Z₁₈) de formule (II) : 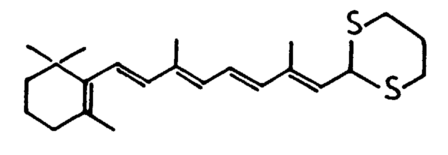
Pour préparer le réactif, on pèse du trichlorure de titane (3 g - 0,019 mole) dans un ballon sec. Le ballon est purgé à l'argon et on ajoute du tétrahydrofuranne anhydre (70 ml), puis 0,5 équivalent de LiAlH₄ (7,5 ml de solution 1,29M dans l'éther). Le milieu est agité pendant 15 minutes à température ambiante.
A l'abri de la lumière, 2,5 équivalents de ce réactif (55 ml de la suspension) sont ajoutés au goutte à goutte et à -78°C à 1 équivalent de composé (Z₁₇) (2,2 g - 5,38 mmole) en solution dans du THF anhydre (50 ml). Après avoir enlevé le bain froid, le milieu est agité pendant 3h à température ambiante, puis hydrolysé lentement à -30°C par addition d'eau (250 ml). La phase aqueuse est extraite à l'éther (4 x 100 ml). Les phases organiques rassemblées sont lavées par une solution saturée en NaCl (100 ml), filtrées sur une petite couche de célite, séchées sur Na₂SO₄, puis concentrées sous vide à température ambiante. Le précipité jaune 1,2 g obtenu est recristallisé dans du méthanol additionné de quelques gouttes d'éther.
Le rendement est de : 60 %
Les caractéristiques du rétinoïde obtenu sont les suivantes :
a) CCM (hexane/acétate d'éthyle : 70/30)
Rf ≃ 0,75
b) Pf : 108-110°C
c) M = 374,66 g
d) La structure a été confirmée par RMN(¹H et¹³C) (200 MHz) (CDCl₃) et par IR.
e) L'analyse élémentaire donne les résultats suivants :

| % | Calculé | Trouvé |
|---|---|---|
| C | 73,74 | 73,73 |
| H | 9,15 | 9,25 |

### EXEMPLE 2 - Préparation du rétinoïde de formule (III)

### A) Préparation du synthon de départ de formule (VIb)

La préparation est effectuée selon le schéma ci-dessous :

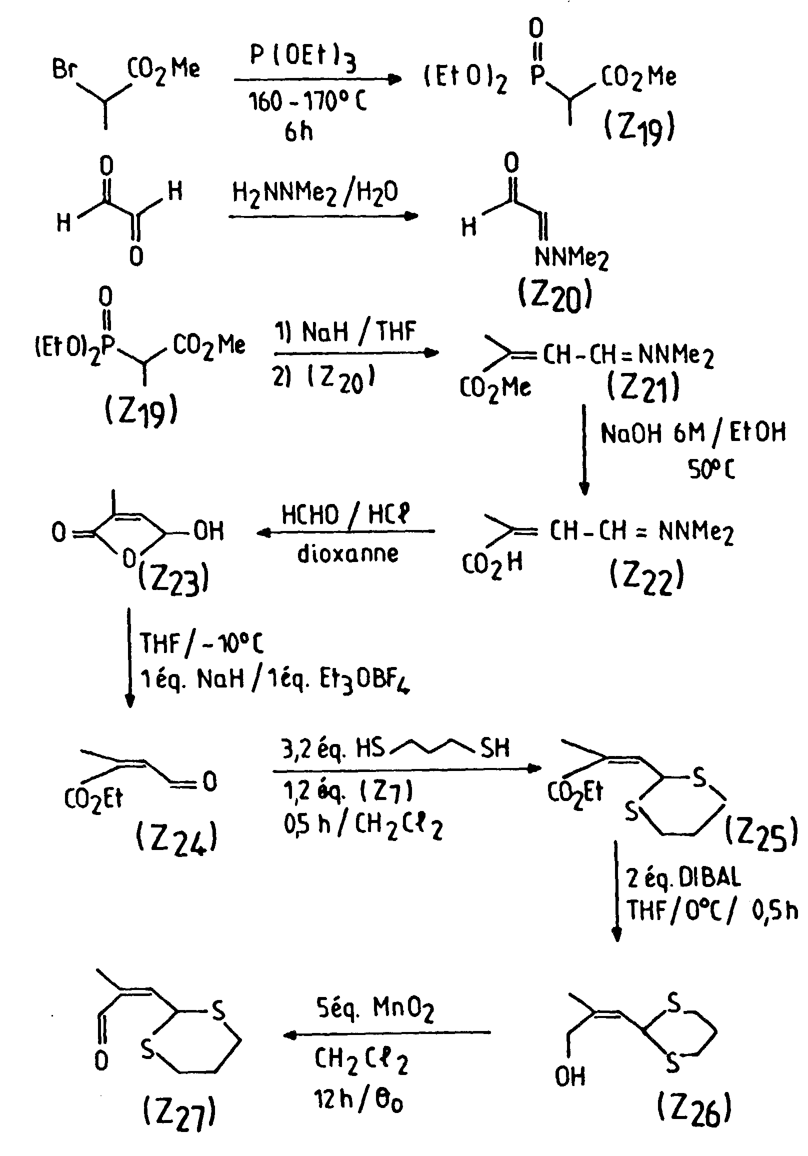

Dans ce schéma, les composés intermédiaires sont indiqués par les références (Zᵢ), i étant un nombre entier allant de 19 à 27 ; θₒ désigne la température ambiante.
1ère étape : Préparation du diéthyl phosphonopropionate de méthyle (Z₁₉) de formule : 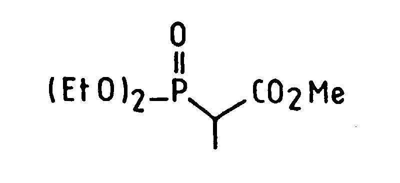 avec : Me = méthyle
Et = méthyle
Un équivalent de 2-bromo propionate de méthyle (22,5 ml - 0,2 mole) est mélangé à 1,5 équivalent de triéthylphosphite (51,5 ml - 0,3 mole). Le milieu est chauffé progressivement à 160 - 170°C. Cette température est maintenue durant 6 heures. Au cours de la réaction, le bromure d'éthyle formé distille (Température ébullition = 40°C) ainsi que de l'acrylate de méthyle (Température ébullition = 80°c). Puis, l'excès de réactif est distillé sous vide (60 - 65°C, sous 66 Pascals). Le phosphonopropionate est distillé (75 - 80°C, sous 66 Pascals) afin d'obtenir 44,84 g d'un liquide incolore.
Le produit obtenu a les caractéristiques suivantes :
a) CCM (hexane/acétate d'éthyle : 70/30)
   Rf ≃ 0,17
b) M = 224,2 g
c) La structure a été confirmée par RMN(¹H et¹³C) (200 MHz) (CDCl₃) et par IR.

2ème étape : Préparation de la mono-N,N-diméthylhydrazone du glyoxal (Z₂₀) de formule : 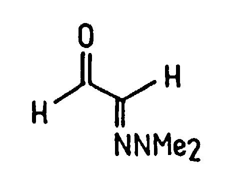 avec Me = méthyle
A 1,1 équivalent de glyoxal (0,5 mole - 29 ml de réactif en solution aqueuse à 30 %) dans de l'eau (300 ml) on ajoute goutte à goutte et sous agitation 1 équivalent de N,N-diméthylhydrazine (34,2 ml - 0,45 mole). Après 0,5 h à température ambiante le milieu est extrait au CH₂Cl₂(3 x 150 ml). Les phases organiques rassemblées sont séchées sur Na₂SO₄ , puis concentrées. Le liquide jaune clair (45 g) obtenu est utilisé tel quel.
Le produit obtenu a les caractéristiques suivantes :
a) CCM (hexane/acétate d'éthyle : 70/30)
   Rf ≃ 0,23
b) M = 100,12 g
c) La structure a été confirmée par RMN(¹H) (200 MHz) (CDCl₃) et en IR.

3ème étape : Préparation de N,N-diméthylhydrazone-4 méthyl-2 butène-2 oate de méthyle (Z₂₁) de formule : 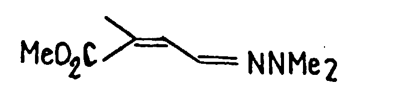 avec Me = méthyle
L'hydrure de sodium (1,07 g - 0,0446 mole) est mis en suspension dans du tétrahydrofuranne (THF) anhydre (40 ml). Le milieu est refroidi à 0°c, puis 1 équivalent du phosphonoacétate (Z₁₉) est ajouté au goutte à goutte sous agitation (10 g - 0,0446 mole). Après 5 minutes, 1 équivalent de l'hydrazone (Z₂₀) (4,46 g - 0,0446 mole) est additionné lentement au milieu. Une gomme jaune brune se dépose rapidement au fond du ballon réactionnel. La réaction est suivie par chromatographie en couche mince jusqu'à disparition complète des produits de départ (15 à 30 minutes). Le milieu est alors hydrolysé par addition d'eau (10 ml), puis extrait à l'éther (3 x 50 ml). Les phases organiques rassemblées sont lavées par une solution saturée en NaCl (50 ml), séchées sur Na₂SO₄, puis concentrées. Le résidu est recristallisé dans un mélange acétone/pentane ; s'il est trop impur, une flash-chromatographie est nécessaire (éluant = hexane/acétate d'éthyle : 80/20 ; silice traitée avec 3 % de triéthylamine). On obtient 7,21 g de produit après recristallisation.
Le rendement est de : 95 %
Les caractéristiques du produit obtenu sont les suivantes :
a) CCM (hexane/acétate d'éthyle : 70/30)
Rf ≃ 0,56
b) Pf = 49-50°C
c) M = 170,2 g
d) La structure a été confirmée par RMN(¹H et¹³c) (200 MHz) (CDCl₃) et par IR.
e) L'analyse élémentaire donne les résultats suivants :

| % | Calculé | Trouvé |
|---|---|---|
| C | 56,46 | 56,28 |
| H | 8,29 | 8,40 |
| N | 16,46 | 16,40 |

4ème étape : Préparation de l'acide N,N-diméthylhydrazone-4 méthyl-2 butène-2 oïque(Z₂₂) de formule : 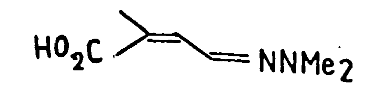 avec Me = méthyle
L'ester (Z₂₁) (10 g - 0,059 mole) est dissous dans un mélange 1/1 d'éthanol et de soude 6M (800 ml). Le mélange réactionnel est alors agité à 50°C jusqu'à disparition du produit de départ (30 min.). Il est ensuite dilué par addition d'eau (100 ml), et l'essentiel de l'alcool est évaporé sous vide. Le résidu est extrait une première fois à l'éther (2 x 100 ml) afin d'enlever tous les produits de dégradation organiques. Après une nouvelle addition d'éther (200 ml) à la phase aqueuse, celle-ci est acidifiée par addition lente d'acide sulfurique concentré en contrôlant le pH. Dès qu'un pH de 3,4 est atteint, le mélange réactionnel est extrait à l'éther (3 x 100 ml), séché sur Na₂SO₄, puis concentré. Le résidu est recristallisé dans un mélange acétone/pentane. On obtient 8,75 g de produit sous forme de longues aiguilles jaunes pâles.
Le rendement est de : 95 %
Les caractéristiques du produit obtenu sont les suivantes :
a) CCM (hexane/acétate d'éthyle : 70/30)
Rf ≃ 0,07
b) Pf = 145-147°c
c) M = 156,19 g
d) La structure a été confirmée par RMN(¹H et¹³c) (200 MHz) (CDCl₃) et par IR.
e) L'analyse élémentaire donne les résultats suivants :

| % | Calculé | Trouvé |
|---|---|---|
| C | 53,83 | 54,00 |
| H | 7,74 | 7,73 |
| N | 17,94 | 18,19 |

5ème étape : Préparation de la 5-hydroxy 3-méthyl 2-(5H)-furanone (Z₂₃) de formule : 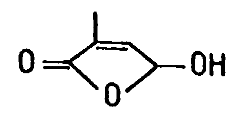
A l'hydrazone (Z₂₂) (6,5 g - 0,0416 mole) en solution dans du dioxane (130 ml), on ajoute 18 ml de formaldéhyde à 35 % dans l'eau et 13 ml d'acide chlorhydrique concentré. Le milieu est agité à température ambiante jusqu'à disparition du produit de départ (5 à 6 h). Puis, il est versé sur de la glace pilée, extrait au chlorure de méthylène (4 x 100 ml), séché sur Na₂SO₄, puis concentré.
Le buténolide obtenu est purifié par flash-chromatographie (éluant = hexane/acétate d'éthyle/éther : 40/30/30). On obtient 3,75 g de cristaux beiges.
Le rendement est de : 79 %
Les caractéristiques du produit obtenu sont les suivantes :
a) CCM (hexane/acétate d'éthyle : 50/50)
Rf ≃ 0,29
b) Pf = 70-72°C
c) M = 114,1 g
d) La structure a été confirmée par RMN(¹H et¹³C) (200 MHz) (CDCl₃) et par IR.
e) L'analyse élémentaire donne les résultats suivants :

| % | Calculé | Trouvé |
|---|---|---|
| C | 52,63 | 52,82 |
| H | 5,30 | 5,33 |

6ème étape : Préparation du tétrafluoroborate de triéthyloxonium (Et₃OBF₄)
A du trifluoroborure éthérate fraîchement distillé (30 ml - 0,243 mole) en solution dans de l'éther anhydre (60 ml) sous argon, est ajouté au goutte à goutte et sous forte agitation 0,75 équivalent d'épichlorhydrine (14,2 ml 0,182 mole) de telle façon qu'il y ait reflux spontané. Ce reflux est maintenu durant 1 h, puis le milieu est laissé au repos durant la nuit. Un gel translucide se dépose au fond du ballon réactionnel. Il est filtré sous argon, lavé abondamment à l'éther anhydre, puis séché à la pompe à palettes. Un précipité blanc de 26 g de tétrafluoroborate de triéthyloxonium est obtenu. Il peut être conservé plusieurs jours au réfrigérateur sous argon.
Le rendement est de : 78 %
La masse molaire est M = 183,94 g
7ème étape : Préparation de β-formyl méthacrylate d'éthyle sous forme -cis (Z₂₄) de formule : 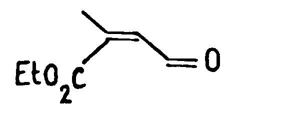 avec Et = éthyle
Le buténolide (Z₂₃) (3 g - 0,026 mole) est solubilisé dans du chlorure de méthylène distillé (60 ml). Après refroidissement du milieu à -10°C, 1 équivalent d'hydrure de sodium (0,624 g - 0,026 mole) est additionné, suivi immédiatement d'l équivalent de tétrafluoroborate de triéthyloxonium (4,97 g - 0,026 mole). Le milieu est agité pendant 1,5 h de -10°c à environ 5°C, avant d'être hydrolysé par addition d'un équivalent de triéthylamine (3,6 ml - 0,026 mole) et de 30 ml d'éther anhydre. Le précipité jaune pale formé (complexe de la triéthylamine et du trifluoroborure) est filtré et lavé abondamment à l'éther anhydre. Le résidu est évaporé, puis traité par chromatographie sur colonne de silice (éluant = hexane/acétate d'éthyle : 80/20 silice traitée avec 3 % de triéthylamine). On obtient 2,22 g d'une huile jaune. Le rendement est de : 60 %
Les caractéristiques du produit sont les suivantes :
a) CCM (hexane/acétate d'éthyle : 70/30)
Rf ≃ 0,62
b) M = 142,16 g
c) La structure a été confirmée par RMN(¹H et¹³C) (200 MHz) (CDCl₃) et par IR.
d) L'analyse élémentaire donne les résultats suivants :

| % | Calculé | Trouvé |
|---|---|---|
| C | 59,14 | 58,88 |
| H | 7,09 | 7,21 |

8ème étape : Préparation du β-(1,3-dithiane) méthacrylate d'éthyle sous forme -cis (Z₂₅) de formule : 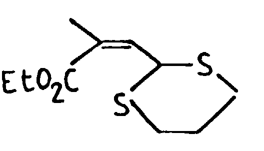 avec Et = éthyle
On met en solution 3,2 équivalents de 1,3-propane dithiol (4,85 ml - 0,048 mole) et 1,2 équivalent de Zn(OTf)₂ (composé (Z₇) obtenu comme indiqué dans l'exemple 1) (6,54 g - 0,018 mole) dans du CH₂Cl₂ distillé (80 ml) sous argon. Après agitation du milieu pendant 15 minutes à température ambiante, 1 équivalent de l'aldéhyde (Z₂₄ (2,2 g - 0,015 mole) en solution dans du CH₂Cl₂ (20 ml) est additionné au goutte à goutte rapide. La réaction est complète après 0,5 h et le milieu est dilué par addition d'eau (90 ml), puis d'une solution saturée en NH₄Cl jusqu'à ce que l'on atteigne un pH compris entre 4 et 5 (environ 200 ml). La phase aqueuse est extraite par un mélange hexane/éther (1/1) (4 x 100 ml). Les phases organiques rassemblées sont lavées par une solution saturée en NH₄Cl (100 ml) afin de "casser" l'émulsion qui s'est formée, puis par de la soude 0,5M (3 x 100 ml), par une solution saturée en NaHCO₃(100 ml), par de l'eau (2 x 100 ml) et enfin par une solution saturée en NaCl (100 ml). Après séchage sur Na₂SO₄ et évaporation du solvant, 3,6 g d'une huile incolore sont isolés.
Les caractéristiques du produit obtenu sont les suivantes :
a) CCM (hexane/acétate d'éthyle : 90/10)
Rf ≃ 0,36
b) M = 232,37 g
c) La structure a été confirmée par RMN(¹H et¹³C) (200 MHz) (CDCl₃) et par IR.
d) L'analyse élémentaire donne les résultats suivants :

| % | Calculé | Trouvé |
|---|---|---|
| C | 51,69 | 51,83 |
| H | 6,94 | 6,85 |

9ème étape : Préparation du composé (Z) 3-(1,3-dithiane 2 yl )2-méthylpropenol (Z₂₆) de formule : 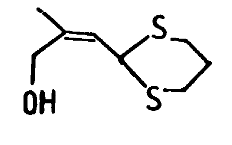
L'ester (Z₂₅) (3,5 g - 0,015 mole) est mis en solution dans du tétrahydrofuranne (THF) anhydre (70 ml) sous argon. Après refroidissement du milieu à 0°c, 2,2 équivalents d'hydrure de diisobutylammonium (DIBAL) (33 ml de solution 1M dans le toluène) sont additionnés au goutte à goutte rapide. Après O,5h,la réaction est complète et le milieu est hydrolysé par addition lente de méthanol (1,55 ml). Le tout est versé sur un mélange d'acétate d'éthyle (600 ml) et d'une solution saturée en tartrate de sodium (74 ml). Une agitation vigoureuse de ce milieu durant 1h permet de "casser" le gel qui s'est formé. La phase aqueuse est alors extraite à l'acétate d'éthyle (3 fois 100 ml). Les phases organiques rassemblées sont lavées avec une solution saturée en NaCl (100 ml), séchées sur Na₂SO₄, puis concentrées. Le résidu est traité par chromatographie sur colonne de silice (éluant = hexane/acétate d'éthyle : 80/20 ; silice traitée avec 3 % de triéthylamine) pour donner 2,30 g d'une huile incolore.
Le rendement est de : 81 %
Le produit obtenu a les caractéristiques suivantes :
a) CCM (hexane/acétate d'éthyle : 70/30)
Rf ≃ 0,23
b) La structure a été confirmée par RMN(¹H et ¹³C) (200 MHz) (CDCl₃) ) et par IR.
c) L'analyse élémentaire donne les résultats suivants :

| % | Calculé | Trouvé |
|---|---|---|
| C | 50,49 | 50,69 |
| H | 7,41 | 7,69 |

10ème étape : Préparation du synthon constitué par du (Z) 3-(1,3-dithiane 2-yl) 2-méthylpropenal (Z₂₇) de formule : 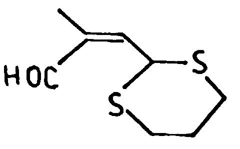
L'alcool (Z₂₆) (2,3 g - 0,012 mole) est dissous dans du CH₂Cl₂ distillé (150 ml) sous argon. Environ 5 équivalents de dioxyde de manganèse (5,25 g) sont ajoutés au milieu sous vigoureuse agitation. Après 4h à température ambiante, la suspension est filtrée sur une petite couche de silice. La silice est abondamment lavée à l'acétate d'éthyle. Le filtrat est concentré sous vide. On obtient 2,15 g de cristaux beiges.
Le rendement est de : 95 %
Les caractéristiques du produit sont les suivantes :
a) CCM (hexane/acétate d'éthyle : 70/30)
Rf ≃ 0,52
b) Pf = 102°C
c) M = 188,32 g
d) La structure a été confirmée par RMN(¹H et¹³C) (200 MHz) (CDCl₃) et par IR.
e) L'analyse élémentaire donne les résultats suivants :

| % | Calculé | Trouvé |
|---|---|---|
| C | 51,02 | 51,20 |
| H | 6,42 | 6,47 |

### B) Préparation du rétinoïde de formule (III)

La préparation est effectuée selon le schéma réactionnel suivant :

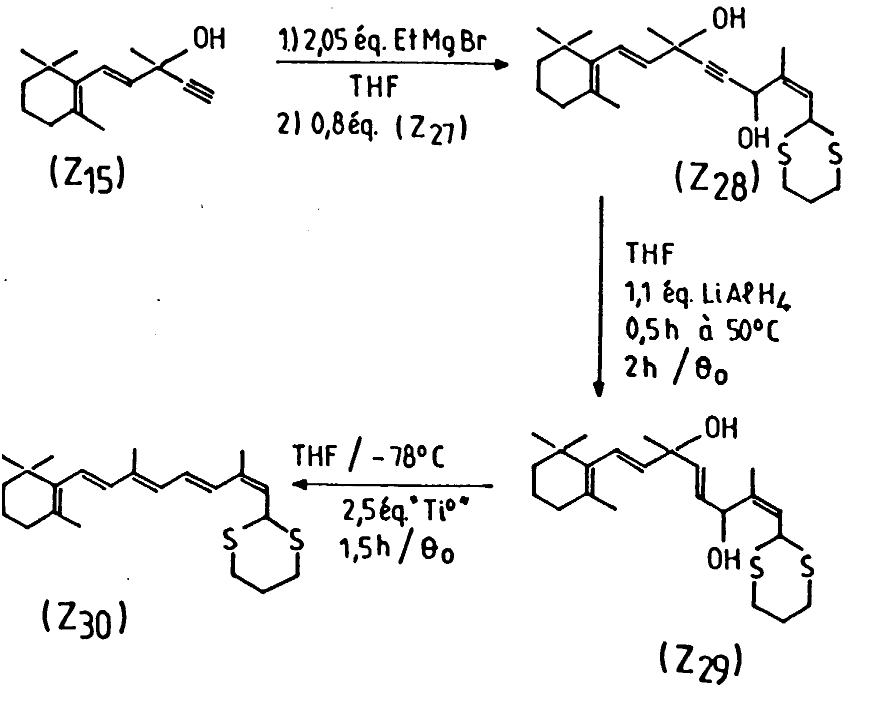

Le composé (Z₁₅) est préparé comme décrit dans l'exemple 1.

Dans ce schéma, les composés intermédiaires sont indiqués par les références (Zᵢ), i étant un nombre entier allant de 28 à 30.θₒ désigne la température ambiante.
1ère étape : Préparation du composé (Z₂₈) de formule : 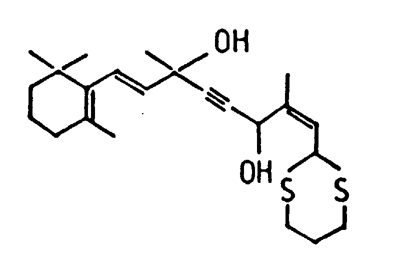
   Dans un premier temps, un réactif de Grignard est préparé à partir de 2,05 équivalents de magnésium (79,2 mg - 3,26 mmole) dans du tétrahydrofuranne (THF) anhydre (10 ml) et sous argon. 2,1 équivalents de bromoéthane (0,25 ml - 3,34 mmole) en solution dans du THF anhydre (10 ml) sont ajoutés au goutte à goutte. Dès que le magnésium est dissous, 1 équivalent du composé (Z₁₅) (348 mg - 1,59 mmole) en solution dans du THF (10 ml) est additionné lentement. Un dégagement d'éthane est observé. Après 2 h de réaction à température ambiante, 0,8 équivalent du composé (Z₂₇) (0,24 g-1,275 mmole) en solution dans du THF (10 ml) est ajouté au milieu rapidement. Après 1,5 h, la teneur en composé (Z₂₈) n'évoluant plus comme on le constate par chromatographie en couche mince, le milieu est hydrolysé par addition lente d'une solution saturée en NH₄Cl (20 ml ; pH = 7), puis d'eau (10 ml). La phase aqueuse est extraite à l'éther (3 x 50 ml). Les phases organiques rassemblées sont lavées avec une solution saturée en NaCl (80 ml), séchées sur Na₂SO₄, puis concentrées. Le résidu est purifié par flash-chromatographie (éluant = hexane/acétate d'éthyle : 80/20). On obtient 430 mg d'une huile jaune très "mousseuse", qui peut être conservée plusieurs mois au congélateur, mais elle se dégrade très vite à température ambiante.
   Le rendement est de : 83 % par rapport au composé (Z₂₇).
   Les caractéristiques du produit obtenu sont les suivantes :
   a) CCM (hexane/acétate d'éthyle : 70/30)
      Rf ≃ 0,24
   b) M = 406,66 g
   c) La structure a été confirmée par RMN(¹H) (200 MHz) (CDCl₃) et étude du spectre infrarouge (CCl₄).
2ème étape : Préparation du composé (Z₂₉) de formule : 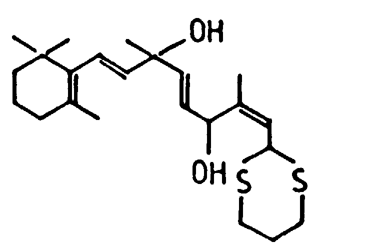
   A 1 équivalent du composé (Z₂₈) (350 mg - 0,86 mmole) en solution dans du tétrahydrofuranne (THF) anhydre (35 ml) sous argon, on ajoute au goutte à goutte 1,4 équivalent de LiAlH₄ (1,1 ml de solution 0,89 M dans l'éther). Quand l'addition est terminée, le ballon réactionnel équipé d'un réfrigérant est plongé dans un bain préalablement chauffé à 50°C. A cette température, le milieu jaune fonce peu à peu. Après 1,5 h, le milieu se dégradant trop, l'agitation est poursuivie à température ambiante pendant 2 h. Puis le milieu est prudemment hydrolysé par addition d'une solution saturée en NH₄Cl (20 ml ; pH = 7), puis d'eau (10 ml). La phase aqueuse est extraite à l'éther (3 x 20 ml). Les phases organiques rassemblées sont lavées par une solution saturée en NaCl (50 ml), séchées sur Na₂SO₄, puis concentrées. Le résidu est purifié par flash-chromatographie (éluant = hexane/ acétate d'éthyle : 90/10). On obtient 105mg d'une huile jaune ; cette huile peut être conservée difficilement au congélateur et se dégrade très vite à température ambiante.
   Le rendement est de : 55 %
   Le produit obtenu a les caractéristiques suivantes :
   a) CCM (hexane/acétate d'éthyle : 70/30)
      Rf ≃ 0,23
   b) M = 408,67 g
   c) La structure a été confirmée par RMN(¹H) (200 MHz) (CDCl₃)
3ème étape : Préparation du rétinoide (Z₃₀) de formule (III) : 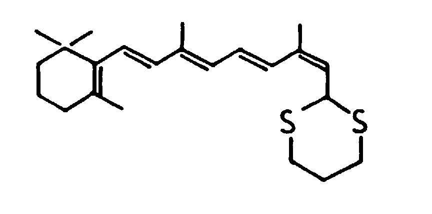
   Pour préparer le réactif contenant le titane basse valence (Ti°),le trichlorure de titane (1,5 g - 9,72 mmole) est pesé dans un ballon sec équipé d'un réfrigérant ; le ballon est purgé à l'argon et du tétrahydrofuranne (THF) anhydre (48 ml) y est ajouté, suivi de 0,5 équivalent de LiAlH₄ (2,2 ml de solution 2,2 M dans l'éther). Le milieu est agité pendant 10 minutes à température ambiante puis on y ajoute 0,42 équivalent de triéthylamine (0,57 ml - 4,08 mmole) et le tout est chauffé à reflux pendant 1,5 h.
   A l'abri de la lumière, 2,1 équivalents du réactif obtenu (3,7 ml de la suspension) est ajouté au goutte à goutte et à -78°C à 1 équivalent de composé Z₂₉ (0,14mg - 0,343 mmole) en solution dans du THF anhydre (20 ml). Le milieu est alors plongé dans un bain à 50°C et agité 0,5 h à cette température, puis hydrolysé lentement à -30°C par addition d'eau (10 ml). La phase aqueuse est extraite à l'éther (4 x 15 ml). Les phases organiques rassemblées sont filtrées sur une petite couche de célite, séchées sur Na₂SO₄, puis concentrées sous vide. Le résidu obtenu est purifié par flash-chromatographie (éluant : hexane/ éther : 95/5). On obtient 64 mg de cristaux jaunes.
   Le rendement est de : 50 %.
   Le produit obtenu a les caractéristiques suivantes :
   a) CCM (hexane/acétate d'éthyle : 70/30)
      Rf ≃ 0,80
   b) M = 374,66 g
   c) La structure a été confirmée par RMN(¹H) (200 MHz) (CDCl₃)

### EXEMPLE 3 : Préparation du rétinoïde de formule IV :

### A) Préparation du synthon de formule (VIc) :

La préparation est effectuée selon le schéma suivant :

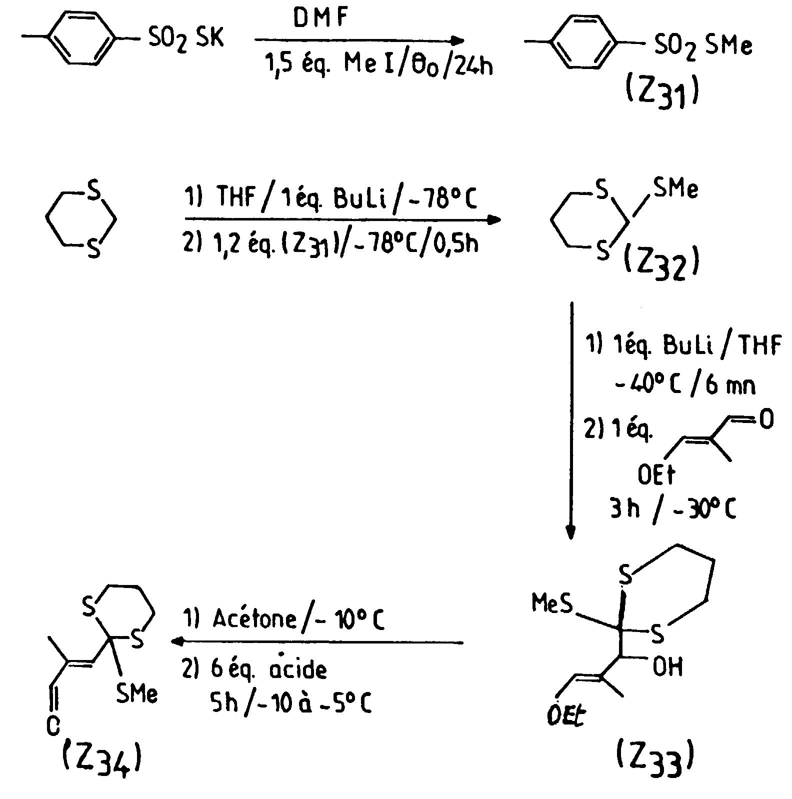

Dans ce schéma, les composés intermédiaires sont indiqués par les références (Zᵢ), i étant un nombre entier allant de 31 à 34 ; θₒ désigne la température ambiante.
1ère étape : Préparation du S-méthyl 4-méthylbenzènethiosulfonate (Z₃₁) de formule : 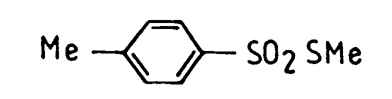 avec Me = méthyle
A 1 équivalent de toluylthiosulfonate de potassium (35 g - 0,155 mole) en solution dans le diméthylformamide distillé sur tamis moléculaire 4 Angströms (500 ml), on ajoute 1,3 équivalent d'iodométhane (12,8 ml - 0,201 mole) au goutte à goutte rapide. Le milieu est agité pendant 24 h à température ambiante. En cours de réaction, il brunit peu à peu. Après dilution par addition d'eau (450 ml), le milieu est extrait au CH₂Cl₂ (5 x 150 ml). Les phases organiques rassemblées sont lavées par une solution saturée en NaHCO₃ (2 x 150 ml) par une solution saturée en Na₂SO₃ (200 ml) ce qui permet de décolorer le milieu, puis lavées abondamment a l'eau (5x 200 ml). Après un dernier lavage par une solution saturée en NaCl (250 ml) suivi d'un séchage sur Na₂SO₄ et d'une concentration, un précipité jaune est obtenu. Il est recristallisé dans de l'éther additionné d'un peu de pentane. On obtient 27,91 g de gros cristaux blancs.
Le rendement est de : 89 %
Les caractéristiques du produit obtenu sont les suivantes :
a) CCM (hexane/acétate d'éthyle : 70/30)
   Rf ≃ 0,5
b) Pf : 58-59°C
c) M = 202,3 g
d) La structure a été confirmée par RMN (¹H et¹³C) (200 MHz) (CDCl₃) et par IR.

2ème étape : Préparation du 2-méthylthio 1,3-dithiane (Z₃₂) de formule : 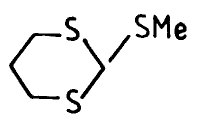 avec Me = méthyle
Une solution de 1,3-dithiane (6 g - 0,05 mole) dans du tétrahydrofuranne (THF) anhydre (200 ml) est refroidie à -78°C ; 1 équivalent de n-butyl lithium (33,3 ml de solution 1,5 M) est ajouté au goutte à goutte rapide. Le milieu est agité pendant 2 h à cette température ; puis il est ajouté à l'aide d'une canule sur 1,2 équivalent de composé (Z₃₁) (12,14 g - 0,06 mole) en solution dans du THF anhydre (80 ml) à -78°C. Lors de l'addition (qui a lieu en 35 minutes environ), un précipité blanc se forme progressivement. L'agitation est maintenue à -78°c durant 0,5 h, puis le milieu est hydrolysé par addition d'HCl 0,05 N (600 ml). Le THF est évaporé, puis le résidu est extrait par un mélange CH₂Cl₂/pentane (1/1) (4 x 200 ml). Les phases organiques rassemblées sont lavées par une solution saturée en NaHcO₃ (3 x 100 ml), par une solution saturée en NaCl (250 ml), puis séchées sur Na₂SO₄ et concentrées. Le résidu est rapidement soumis à une chromatographie sur colonne de silice (éluant = hexane/éther : 95/5) afin d'enlever l'excès de toluylthiosulfonate de méthyle (Z₃₁). On obtient 7,90 g d'une huile incolore qui précipite au congélateur.
Le rendement est de : 95 %
Les caractéristiques du produit obtenu sont les suivantes :
a) CCM (hexane/éther : 90/10)
   Rf ≃ 0,54
b) M = 166,33 g
c) La structure a été confirmée par RMN(¹H et¹³C) (200 MHz) (CDCl₃)

3ème étape : Préparation du [3-(2-méthylthio 1,3-dithiane 2-yl) 3-hydroxy 2-méthyl 1-propenyl]éther d'éthyle (Z₃₃) de formule : 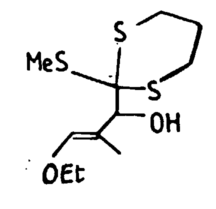 avec Me = méthyle
Et = éthyle
A l'orthothioester (Z₃₂) (7,5 g - 0,045 mole) en solution dans du tétrahydrofuranne (THF) distillé (150 ml) à -40°C, est additionné au goutte à goutte rapide 1 équivalent de n-butyl lithium (30 ml de solution 1,5 M). Après 6 minutes de réaction à cette température, 1 équivalent d'éthoxyméthacroléine (5,35 ml - 0,045 mole) est ajouté. L'agitation est maintenue pendant 3 h à -30°C, puis le milieu est hydrolysé par addition d'une solution saturée en NH₄Cl (environ 200 ml - pH = 5). Dès que la température est remontée à l'ambiante, on extrait à l'éther (3 x 200 ml). Les phases organiques rassemblées sont lavées par une solution saturée en NaHCO₃ (2 x 200 ml), de l'eau (200 ml), une solution saturée en NaCl (200 ml), puis séchées sur Na₂SO₄ et concentrées. Le résidu jaune est purifié par flash-chromatographie (éluant = hexane/éther : 90/10). On obtient 10,25 g d'une huile jaune claire que l'on conserve au congélateur où elle précipite.
Le rendement est de : 81 %
Le produit obtenu a les caractéristiques suivantes :
a) CCM (hexane/éther : 90/10)
Rf ≃ 0,12
b) Pf : 39-40°C
c) M = 280,48 g
d) La structure a été confirmée par RMN (¹H et ¹³C) (200 MHz) (CDCl₃) et par IR.
e) L'analyse élémentaire donne les résultats suivants

| % | Calculé | Trouvé |
|---|---|---|
| C | 47,11 | 47,37 |
| H | 7,19 | 7,22 |

4ème étape : Préparation de (E) 3-(2-méthylthio 1,3-dithiane 2-yl) 2-méthylpropenal (Z₃₄)de formule : 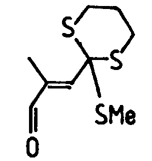 avec Me = méthyle
L'éther d'énol (Z₃₃) (9 g - 0,032 mole) est mis en solution dans de l'acétone (300 ml) à -10°C ; 6 équivalents d'acide (54 ml d'acide sulfurique à 10 % dans l'eau) sont ajoutés lentement au milieu. Celui-ci est agité pendant 5 h entre -10 et -5°C avant d'être hydrolysé par addition d'une solution saturée en NaHCO₃ (environ 200 ml ; pH = 5) et d'eau (200 ml). L'acétone est évaporé, puis le milieu est extrait à l'éther (5 x 100 ml).
Les phases organiques rassemblées sont lavées par une solution saturée en NaHCO₃ (70 ml ; pH = 7) et une . solution saturée en NaCl (250 ml). Après séchage sur Na₂SO₄ et concentration, le résidu est purifié par flash-chromatographie (éluant hexane/éther : 90/10). On obtient 5,62 g d'une huile jaune.
Le rendement est de : 75 %
Les caractéristiques du produit obtenu sont les suivantes :
a) CCM (hexane/éther : 90/10)
Rf ≃ 0,28
b) M = 234,41 g
c) La structure a été confirmée par RMN (¹H et¹³C) (200 MHz) (CDCl₃) et par IR.
d) L'analyse élémentaire donne les résultats suivants :

| % | Calculé | Trouvé |
|---|---|---|
| C | 46,12 | 46,22 |
| H | 6,02 | 5,95 |

### B) Préparation du rétinoïde de formule IV :

La préparation est effectuée selon le schéma ci-dessous.

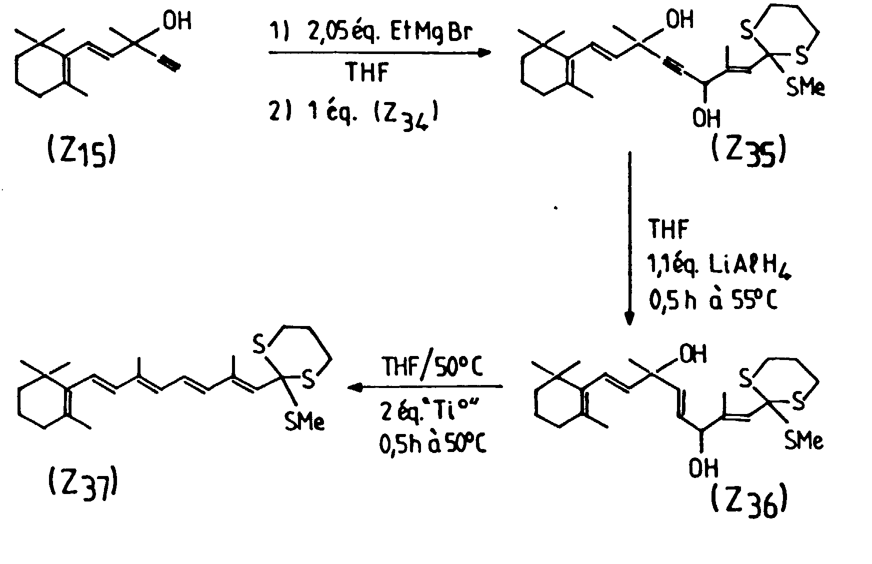

Dans ce schéma, les composés intermédiaires sont indiqués par les références (Zᵢ), i étant un nombre entier allant de 35 à 37.

Le composé acétylénique (Z₁₅) est préparé comme décrit dans l'exemple 1.
1ère étape : Préparation du composé (Z₃₅) de formule : 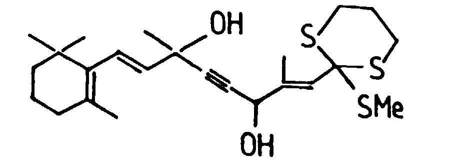 avec Me = méthyle
   Dans un premier temps, un réactif de Grignard est préparé à partir de 2,05 équivalents de magnésium (1,13 g - 0,047 mole) dans du tétrahydrofuranne (THF) anhydre (20 ml) et sous argon. 2,1 équivalents de bromoéthane (3,6 ml - 0,048 mole) en solution dans du THF anhydre (20 ml) sont ajoutés au goutte à goutte. Dès que tout le magnésium est dissous, 1 équivalent du composé (Z₁₅) (5 g - 0,0229 mole) en solution dans du THF (50 ml) est additionné lentement. Un dégagement d'éthane est observé. Après 1,5 h de réaction à température ambiante, 1 équivalent d'aldéhyde (Z₃₄ ) (5,37 g - 0,0229mole) en solution dans du THF (50 ml) est ajouté au milieu rapidement. Après 1 h la teneur en composé (Z₃₅) n'évoluant plus comme on le constate par chromatographie en couche mince,
   le milieu est hydrolysé par addition lente d'une solution saturée en NH₄Cl (50 ml), suivie d'eau (50 ml). La phase aqueuse est extraite à l'éther (3 x 80 ml). Les phases organiques rassemblées sont lavées avec une solution saturée en NaCl (100 ml), séchées sur Na₂SO₄ , puis concentrées. Le résidu est purifié par flash-chromatographie (éluant = hexane/acétate d'éthyle : 85/15). On obtient 9,33 g d'une huile jaune très "mousseuse".
   Le rendement est : 90 %
   Le produit obtenu a les caractéristiques suivantes :
   a) CCM (hexane/acétate d'éthyle : 70/30)
      Rf ≃ 0,29
   b) M = 452,75 g
   c) La structure a été confirmée par RMN (¹H) (200 MHz) (CDCl₃) et étude du spectre IR (CCl₄).
2ème étape : Préparation du diol (Z₃₆) de formule : 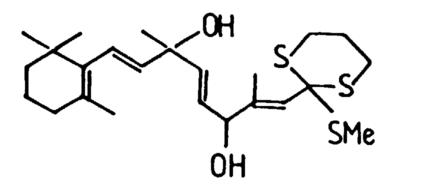 avec Me = méthyle
   A 1 équivalent du composé (Z₃₅) (1,1 g - 2,43 mmole) en solution dans du tétrahydrofuranne (THF) anhydre (140 ml) sous argon est ajouté au goutte à goutte 1,1 équivalent de LiAlH₄ (2,7 ml de solution 1 M dans l'éther). L'addition terminée, le ballon équipé d'un réfrigérant est plongé dans un bain préalablement chauffé à 55°C. A cette température, le milieu jaune prend peu à peu une couleur violette. Après 0,5 h, la réaction est totale et le milieu est prudemment hydrolysé par addition d'une solution saturée en NH₄Cl(60 ml), suivie d'eau (20 ml). La phase aqueuse est extraite à l'éther (3 x 70 ml). Les phases organiques rassemblées sont lavées par une solution saturée en NaCl (100 ml), séchées sur Na₂SO₄, puis concentrées. Le résidu est purifié par flash-chromatographie (éluant = hexane/acétate d'éthyle : 90/10). On obtient 0,9 g d'une huile jaune.
   Le rendement est de : 81 %
   Les caractéristiques du produit obtenu sont les suivantes :
   a) CCM (hexane/acétate d'éthyle : 70/30)
      Rf ≃ 0,21
   b) M = 454,77 g
   c) La structure a été confirmée par RMN(¹H) (200 MHz) (CDCl₃)
3ème étape : Préparation du rétinoïde de formule (IV) : 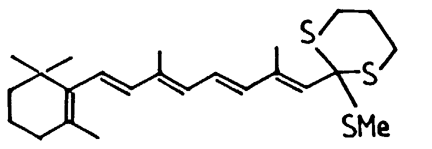 avec Me = méthyle
   Pour préparer le réactif contenant le titane basse valence (Ti°), on pèse du trichlorure de titane (0,5 g - 3,24 mmole) dans un ballon sec équipé d'un réfrigérant. Le montage est purgé à l'argon et du tétrahydrofuranne (THF) anhydre (80 ml) est ajouté. On ajoute ensuite 0,5 équivalent de LiAlH₄ (1,47 ml de solution 1,1 M dans l'éther). Le milieu est agité pendant 10 minutes à température ambiante, puis 0,2 équivalent de triéthylamine (0,09 ml - 0,648 mmole) y est additionné et le tout est chauffé à reflux pendant 1,5 h.
   A l'abri de la lumière, 2 équivalents du réactif contenant le titane (66,4 ml de la suspension) sont ajoutés, au goutte à goutte et à 50°C à 1 équivalent de composé (Z₃₆) (0,6 g - 1,32 mmole) en solution dans du THF anhydre (30 ml). Le milieu est agité pendant 0,5h à cette température, puis il est hydrolysé lentement à -30°C par addition d'eau (90 ml). La phase aqueuse est extraite à l'éther (4 x 80 ml). Les phases organiques rassemblées sont filtrées sur une petite couche de célite, séchées sur Na₂SO₄, puis concentrées sous vide à température ambiante. Le résidu obtenu est purifié par flash - chromatographie (éluant = hexane/éther : 95/5). On obtient 0,82 g d'une huile jaune.
   Le rendement est de 60 %.
   Le produit obtenu a les caractéristiques suivantes :
   a) CCM (hexane/acétate d'éthyle : 70/30)
      Rf ≃ 0,86
   b) M = 420,76 g
   c) La structure a été confirmée par RMN(¹H) (200 MHz) (CDCl₃) et par IR (CCl₄).

### EXEMPLE 4 : Hydrolyse du rétinoïde (Z₁₈) de formule (IV) :

1ère étape : Obtention du t-butyle N-mésitylènesulfonyloxycarbamate (Z₄₀) de formule : 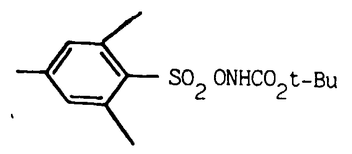 avec t-Bu = tertio-butyl
   A 1 équivalent de chlorure de mésitylène-sulfonyle (5,45 - 0,025 mole)et 1 équivalent de t-butyle N-hydroxycarbamate (3,32 g - 0,025 mole) dissous dans de l'éther anhydre (100 ml) à 0°C, on ajoute au goutte à goutte 1 équivalent de triéthylamine (3,5 ml-0,025 mole). En cours de réaction, un précipité de chlorhydrate de triéthylamine se forme peu à peu. Après 0,5 h d'agitation à 0°C, ce précipité est filtré et lavé abondamment à l'éther. Le milieu est concentré sous vide. On obtient 5,01 g de résidu jaune pale que l'on recristallise 2 fois dans un minimum de benzène chaud (8 ml) et d'hexane (30 ml).
   Le rendement est de 64 %
   La masse moléculaire est :
   M = 315,39 g
2ème étape : Obtention du O-mésitylènesulfonylhydroxylamine (Z₄₁) de formule : 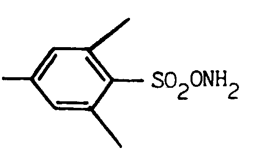
   Le composé (Z₄₀) (3 g - 9,51 mmole) est ajouté à de l'acide trifluoroacétique (11 ml) à 5°C. L'agitation est maintenue pendant 5 minutes à cette température, puis le milieu est versé sur de l'eau glacée (80 ml). Le précipité, qui se forme, est filtré et lavé avec un peu d'eau froide (30 ml). Il est ensuite dissous dans un minimum d'éther (5 ml) et cristallisé par addition d'hexane (15 ml). La cristallisation se fait lentement à froid. Des cristaux blancs sont obtenus.
   Par RMN, on constate la présence d'une forte quantité de produit de départ ; le résidu (2,1 g) a donc été remis en réaction exactement dans les mêmes conditions durant 5 minutes, précipité et recristallisé (0,74 g).
   Le rendement est de 36 %
   La masse moléculaire est :
   M = 315,27 g
3ème étape : Obtention du rétinal tout-trans de formule : 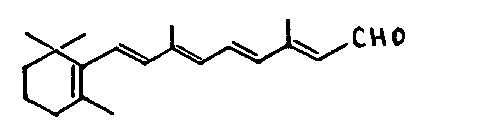
   Le composé (Z₁₈) (64 mg - 1,71.10⁻⁴ mole) en solution dans du CH₂Cl₂(2 ml) est placé à-95°C (pentane + azote liquide), puis 2 équivalents du composé (Z₄₁) (73,5 mg- 3,42.10⁻⁴ mole) en solution dans du CH₂Cl₂ (2 ml) y sont additionnés au goutte à goutte durant 5 minutes. Après 10 minutes à-95°C, le milieu est hydrolysé par addition d'éther (5 ml) et d'une solution saturée en NaCl (10 ml) ; puis on agite vigoureusement durant 0,5 h. La phase aqueuse est extraite à l'éther (2 x 10 ml). Les phases organiques rassemblées sont lavées par une solution saturée en NaCl (2 x 10 ml), séchées sur Na₂SO₄ puis concentrées. Le résidu est purifié par flash-chromatographie (éluant = hexane/acétate d'éthyle : 95/5). Des cristaux jaunes sont obtenus (39 mg).
   Le rendement est de 80 %
   Le produit obtenu a les caractéristiques suivantes :
   a) CCM (hexane/acétate d'éthyle : 70/30)
      Rf ≃ 0,63
   b) M = 284,45 g
Une RMN(¹H) du produit brut réactionnel indique la présence d'un peu d'isomère 13-cis (7 %) qui est éliminé par chromatographie.

### EXEMPLE 5 :

On prépare un gel -en réalisant la formulation suivante
- composé de formule (IV) 0,050 g
- Erythromycine base 4,000 g
- Butylhydroxytoluène 0,050 g
- Hydroxypropylcellulose vendu par la société HERCULES sous le nom de "KLUCEL HF" 2,000 g
- Ethanol (à 95°) qsp 100,000 g

Ce gel est appliqué sur une peau à dermatose ou une peau acnéique 1 à 3 fois par jour. Après 6 à 12 semaines de traitement à raison de 2 à 10 mg par cm² de peau traitée et par application, on observe une amélioration significative.

### EXEMPLE 6 :

On prépare la formulation suivante destinée à être conditionnée dans une gélule :
- composé de formule (IV) 0,060 g
- Amidon de maïs 0,060 g
- Lactose qsp 0,300 g

Les gélules utilisées sont constituées de gélatine, d'oxyde de titane et d'un conservateur.

On administre à un individu adulte 1 à 3 gélules par jour dans le traitement du psoriasis. Après 30 jours de traitement à raison de 1 mg par kg de sujet traité et par jour, on observe une amélioration significative.

### EXEMPLE 7 :

On prépare un gel pour application topique en procédant au mélange des ingrédients suivants :
- composé de formule (II) 0,05 g
- Ethanol 43,00 g
- α-tocophérol 0,05 g
- Acide polyacrylique réticulé vendu par la société "GOODRICH" sous la dénomination "CARBOPOL 940" 0,50 g
- Triéthanolamine (solution aqueuse à 20 %) 3,80 g
- Eau: 9,30 g
- Propylène glycol qsp 100,00 g

Ce gel est appliqué deux fois par jour sur une peau acnéique. Après 6 à 12 semaines de traitement à raison de 2 à 10 mg par cm² de peau traitée et par application, on observe une amélioration significative.

### EXEMPLE 8 :

On prépare un comprimé non soluble en procédant au mélange des substances suivantes :
- composé de formule (III) 0,025 g
- Lactose 0,082 g
- Acide stéarique 0,003 g
- Talc purifié 0,015 g
- Edulcorant qs
- Colorants qs
- Amidon de riz qsp 0,500 g

On administre trois comprimés par jour par voie orale à un individu atteint de psoriasis. Après 30 jours de traitement à raison de 1 mg par kg de sujet traité et par jour, on observe une amélioration significative.

### EXEMPLE 9 :

On réalise un kit anti-acné comprenant deux parties :
a) on prépare un gel ayant la formulation suivante :
   - Alcool éthylique 48,70 g
   - Propylène glycol 50,00 g
   - Acide polyacrylique réticulé vendu par la société "GOODRICH" sous la dénomination "CARBOPOL 940" 1,00 g
   - Butylhydroxyanisole 0,05 g
   - Butylhydroxytoluène 0,05 g
   - α-tocophérol 0,10 g
   - composé de formule (IV) 0,10 g
b) on prépare un gel ayant la formulation suivante :
   - Alcool éthylique 5,00 g
   - Propylène glycol 5,00 g
   - Sel disodique de l'acide éthylène diamine tétracétique 0,05 g
   - Acide polyacrylique réticulé vendu par la société "GOODRICH" sous la dénomination "CARBOPOL 940" 1,00 g
   - Triéthanolamine (99 %) 1,00 g
   - Lauryl sulfate de sodium 0,10 g
   - Eau purifiée 75,05 g
   - Peroxyde de benzoyle hydraté à 25 % 12,80 g

Le mélange des deux gels se fait extemporanément, poids pour poids au moment de l'emploi.

On applique ce mélange 2 fois par jour sur une peau acnéique. Après 4 à 6 semaines de traitement à raison de 2 à 10 mg par cm² de peau traitée et par application, on observe une amélioration significative.

## Revendications (Revendications pour l'(les) Etat(s) contractant(s) suivant(s): AT, BE, CH, DE, DK, FR, GB, IT, LI, NL, PT, SE)

1. Dérivé rétinoïque stéréospécifique de formule : 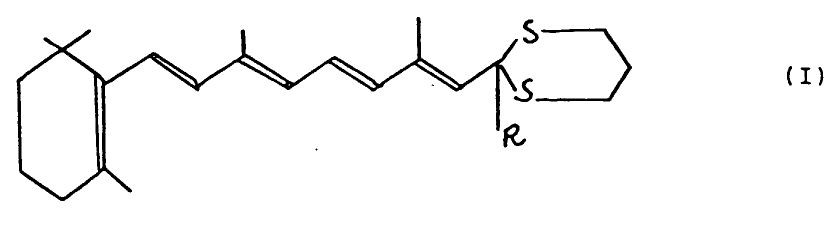 formule dans laquelle R est H ou un radical thioalkyle en C₁ - C₄.

2. Dérivé rétinoïque tout-trans selon la revendication 1, de formule :

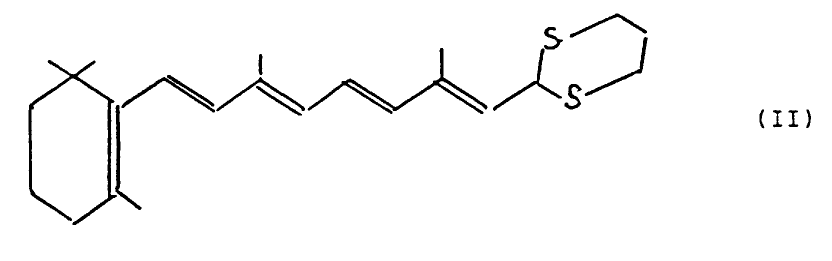

3. Dérivé rétinoïque 13-cis selon la revendication 1, de formule :

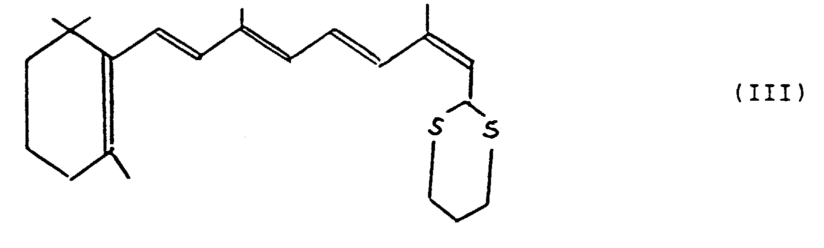

4. Dérivé rétinoïque tout-trans selon la revendication 1, de formule :

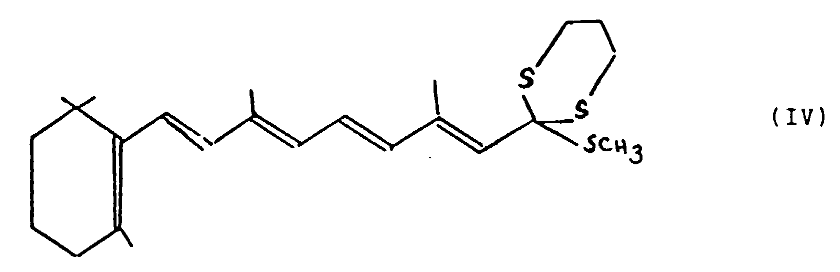

5. Procédé de préparation d'un rétinoïde stéréospécifique selon l'une des revendications 1 à 4, caractérisé par le fait que l'on fait réagir un dithianepropenal de formule : 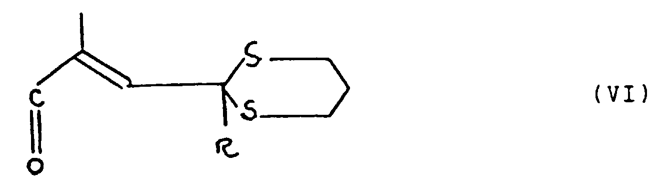 où R a la même signification que dans la revendication 1, avec l'éthyl-β-ionol de formule : 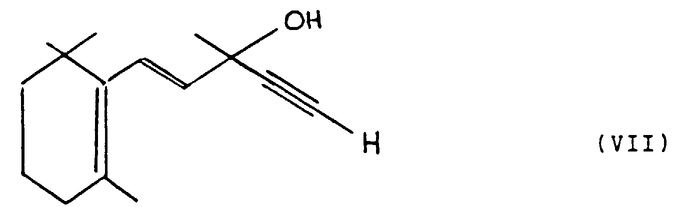 en présence d'un organomagnésien, de façon à obtenir le composé acétylénique de formule : 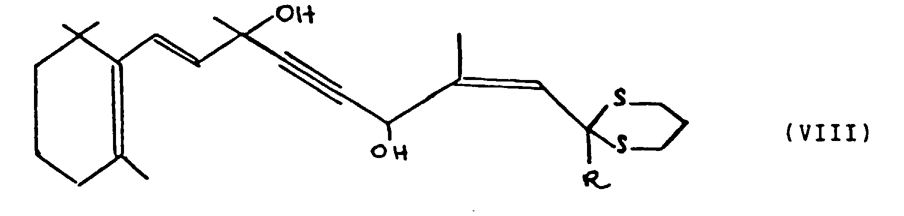 où R a la même signification que précédemment, puis on hydrogène la liaison acétylénique du composé de formule (VIII) pour obtenir le diol de formule : 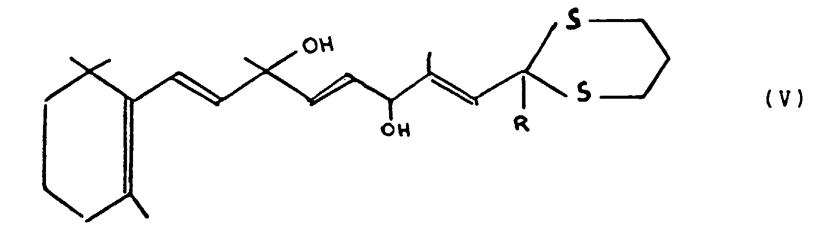 où R a la même signification que dans la revendication 1, et que l'on réduit le diol de formule (V) à l'aide de titane ayant une "basse valence".

6. Procédé selon la revendication 5, caractérisé par le fait que l'hydrogénation du composé de formule (VIII) est effectuée à l'aide d'hydrure de lithium et aluminium.

7. Composition pharmaceutique, caractérisée par le fait qu'elle contient, dans un véhicule approprié pour une administration par voie entérale, parentérale, topique ou oculaire, au moins un composé selon l'une des revendications 1 à 4.

8. Composition selon la revendication 7, caractérisée par le fait qu'elle contient de 0,0001 à environ 5 % en poids, par rapport au poids total de la composition, d'au moins un composé selon l'une des revendications 1 à 4.

9. Utilisation d'une composition pharmaceutique selon l'une des revendications 7 ou 8 pour l'obtention d'un médicament pour le traitement des affections dermatologiques, rhumatismales, respiratoires ou ophtalmologiques.

10. Composition cosmétique pour l'hygiène corporelle et capillaire, caractérisée par le fait qu'elle contient, dans un véhicule cosmétique approprié, au moins un composé selon l'une des revendications 1 à 4.

11. Composition selon la revendication 10, caractérisée par le fait qu'elle contient 0,0001 à environ 3 % en poids, par rapport au poids total de la composition, d'au moins un composé selon l'une des revendications 1 à 4.

## Revendications (Revendications pour l'(les) Etat(s) contractant(s) suivant(s): GR)

1. Dérivé rétinoïque stéréospécifique de formule : 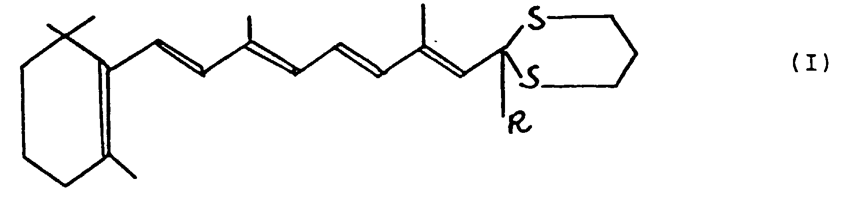 formule dans laquelle R est H ou un radical thioalkyle en C₁ - C₄.

2. Dérivé rétinoïque tout-trans selon la revendication 1, de formule :

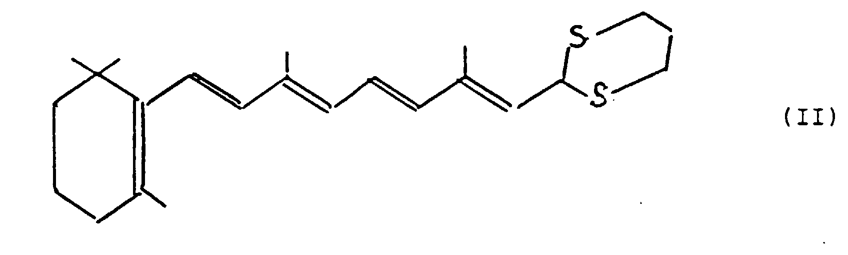

3. Dérivé rétinoïque 13-cis selon la revendication 1, de formule :

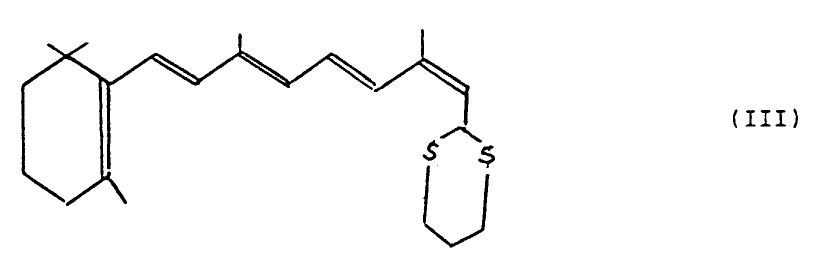

4. Dérivé rétinoïque tout-trans selon la revendication 1, de formule :

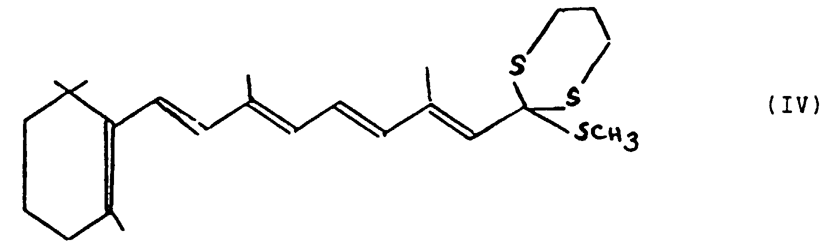

5. Procédé de préparation d'un rétinoïde stéréospécifique selon l'une des revendications 1 à 4, caractérisé par le fait que l'on fait réagir un dithianepropenal de formule : 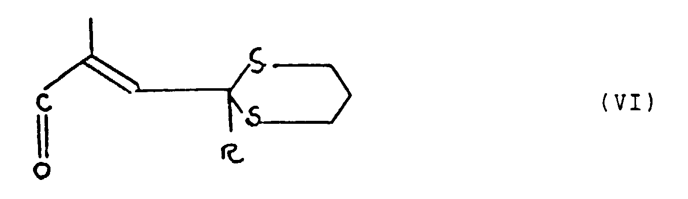 où R a la même signification que dans la revendication 1, avec l'éthyl-β-ionol de formule : 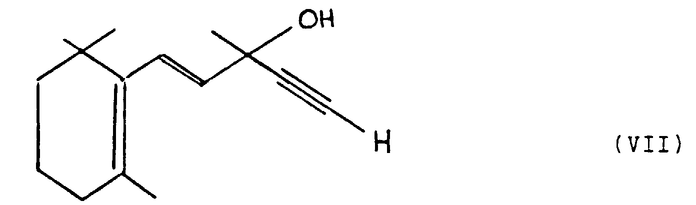 en présence d'un organomagnésien, de façon à obtenir le composé acétylénique de formule : 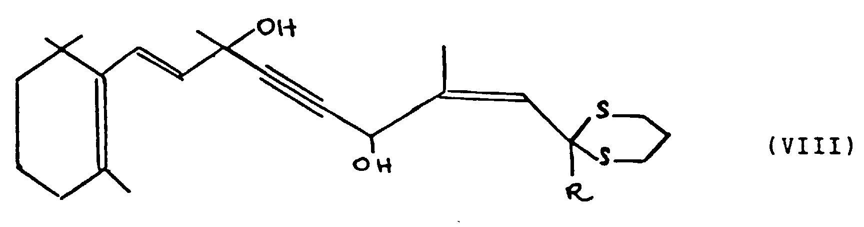 où R a la même signification que précédemment, puis on hydrogène la liaison acétylénique du composé de formule (VIII) pour obtenir le diol de formule : 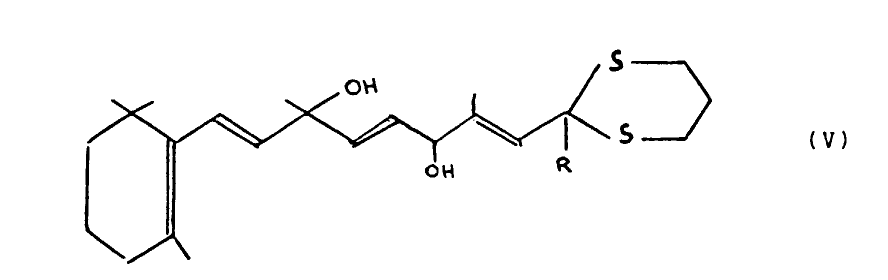 où R a la même signification que dans la revendication 1, et que l'on réduit le diol de formule (V) à l'aide de titane ayant une "basse valence".

6. Procédé selon la revendication 5, caractérisé par le fait que l'hydrogénation du composé de formule (VIII) est effectuée à l'aide d'hydrure de lithium et aluminium.

7. Composition cosmétique pour l'hygiène corporelle et capillaire, caractérisée par le fait qu'elle contient, dans un véhicule cosmétique approprié, au moins un composé selon l'une des revendications 1 à 4.

8. Composition selon la revendication 7, caractérisée par le fait qu'elle contient 0,0001 à environ 3 % en poids, par rapport au poids total de la composition, d'au moins un composé selon l'une des revendications 1 à 4.

## Revendications (Revendications pour l'(les) Etat(s) contractant(s) suivant(s): ES)

1. Procédé de préparation d'un rétinoïde stéréospécifique de formule : 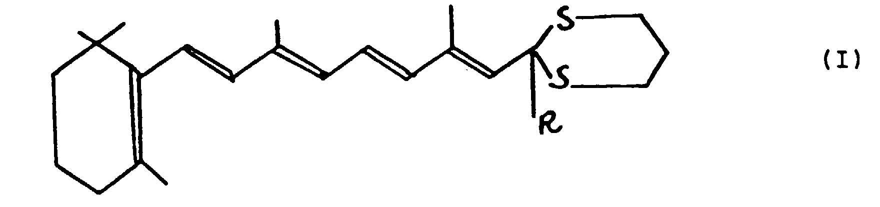 caractérisé par le fait que l'on fait réagir un dithianepropenal de formule : 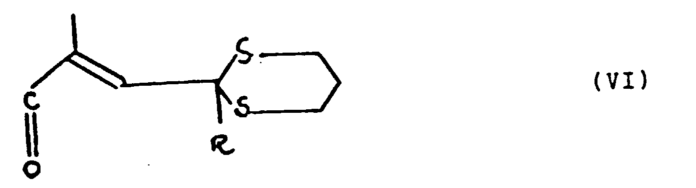 où R est H ou un radical thioalkyle en C₁-C₄, avec l'éthyl-β-ionol de formule : 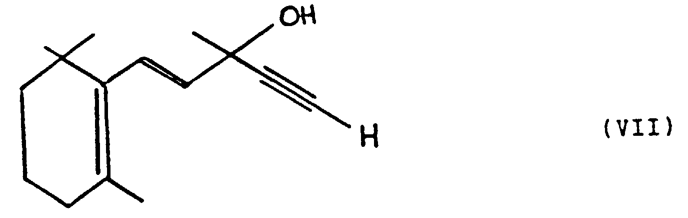 en présence d'un organomagnésien, de façon à obtenir le composé acétylénique de formule : 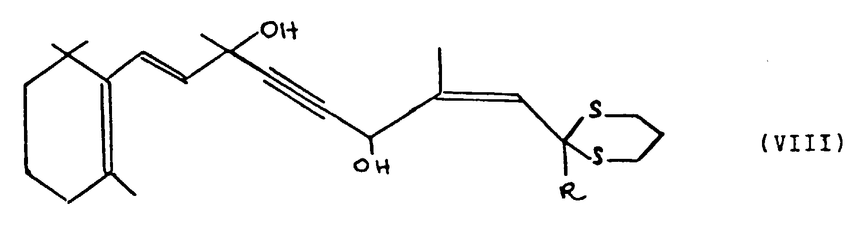 où R a la même signification que précédemment, puis on hydrogène la liaison acétylénique du composé de formule (VIII) pour obtenir le diol de formule : 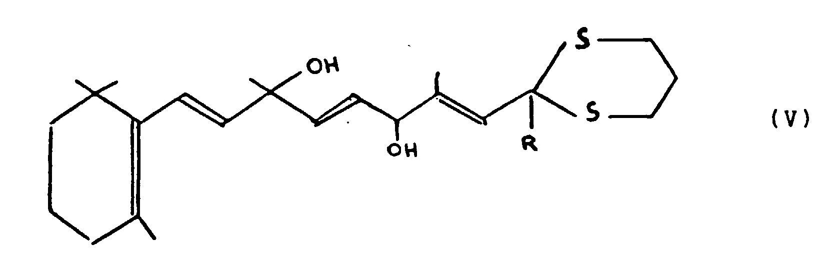 où R est H ou un radical thioalkyle en C₁-C₄, et que l'on réduit le diol de formule (V) à l'aide de titane ayant une "basse valence".

2. Procédé selon la revendication 1, caractérisé par le fait que l'hydrogénation du composé de formule (VIII) est effectuée à l'aide d'hydrure de lithium et aluminium.

3. Composition cosmétique pour l'hygiène corporelle et capillaire, caractérisée par le fait qu'elle contient, dans un véhicule cosmétique approprié, au moins un composé obtenu par le procédé d'une des revendications 1 ou 2.

4. Composition selon la revendication 3, caractérisée par le fait qu'elle contient 0,0001 à environ 3 % en poids, par rapport au poids total de la composition, d'au moins un composé obtenu par le procédé d'une des revendications 1 ou 2.

## Patentansprüche (Patentansprüche für folgende(n) Vertragsstaat(en): AT, BE, CH, DE, DK, FR, GB, IT, LI, NL, PT, SE)

1. Stereospezifisches Retinoid-Derivat der Formel 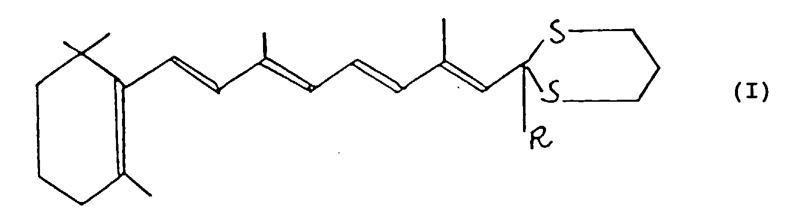 in welcher R für H oder einen C₁-C₄-Thioalkyl-Radikal steht.

2. All-trans-Retinoid-Derivat nach Anspruch 1 der Formel

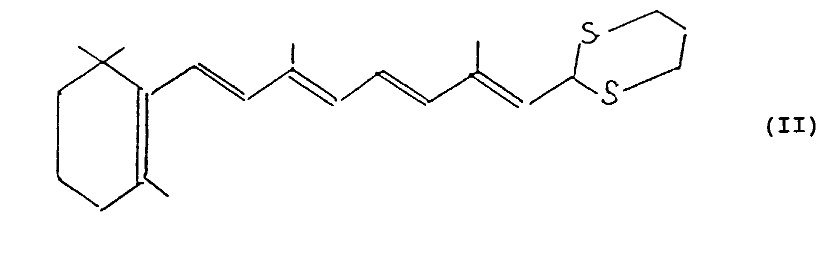

3. 13-cis-Retinoid-Derivat nach Anspruch 1 der Formel

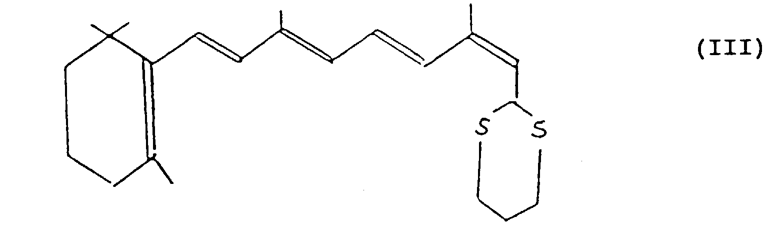

4. All-trans-Retinoid-Derivat nach Anspruch 1 der Formel

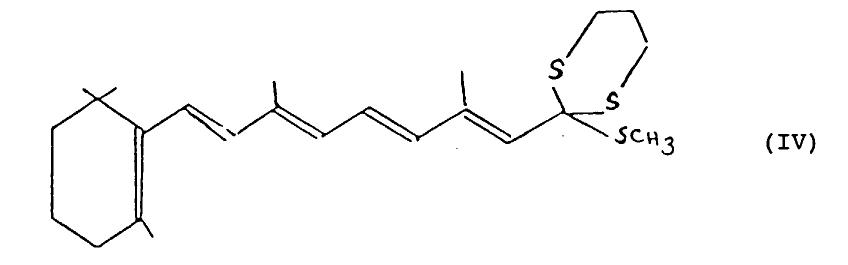

5. Verfahren zur Herstellung eines stereospezifischen Retinoids nach einem der Ansprüche 1 bis 4, dadurch gekennzeichnet, daß man ein Dithianpropenal der Formel 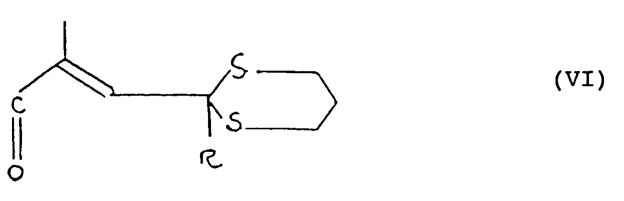 in welcher R dieselbe Bedeutung wie in Anspruch 1 hat, mit Ethy-β-ionol der Formel 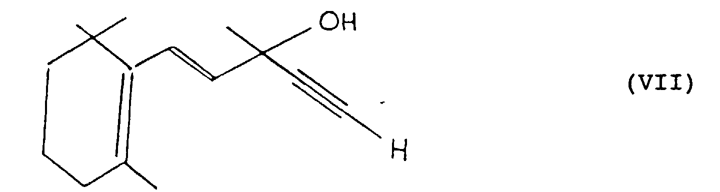 in Anwesenheit eines Organomagnesiums reagieren läßt, so daß man die Acetylenverbindung der Formel 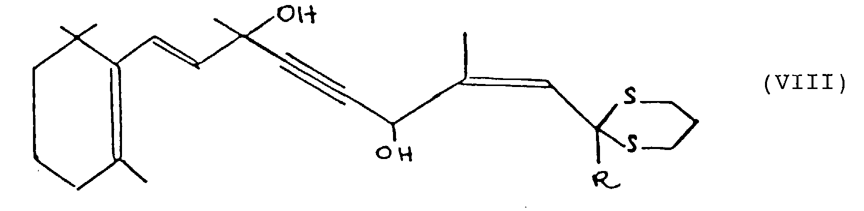 erhält, in der R dieselbe Bedeutung wie oben hat, dann die Acetylenbindung der Verbindung der Formel (VIII) hydrogeniert, um das Diol der Formel: 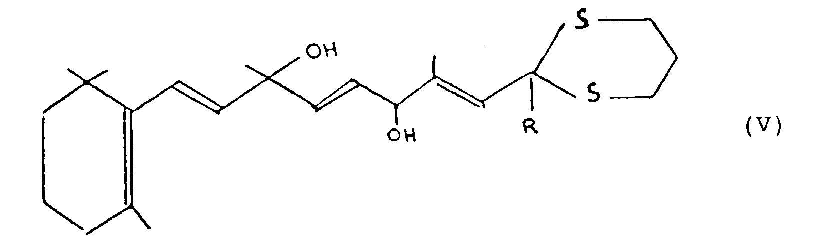 zu erhalten, in der R dieselbe Bedeutung wie in Anspruch 1 hat, und daß man das Diol der Formel (V) mit Hilfe von Titan mit einer "niederen Wertigkeit" reduziert.

6. Verfahren nach Anspruch 5, dadurch gekennzeichnet, daß die Hydrogenierung der Verbindung der Formel (VIII) mit Hilfe von Lithium- und Aluminiumhydrid durchgeführt wird.

7. Pharmazeutische Zusammensetzung, dadurch gekennzeichnet, daß sie in einem für eine enterale, perenterale, topische oder okulare Verabreichung geeigneten Träger mindestens eine Verbindung nach einem der Ansprüche 1 bis 4 enthält.

8. Zusammensetzung nach Anspruch 7, dadurch gekennzeichnet, daß sie, bezogen auf das Gesamtgewicht der Zusammensetzung, 0,0001 bis etwa 5 Gew.-% mindestens einer Verbindung nach einem der Ansprüche 1 bis 4 enthält.

9. Verwendung einer pharmazeutischen Zusammensetzung nach einem der Ansprüche 7 oder 8 zur Herstellung eines Arzneimittels für die Behandlung von dermatologischen und rheumatischen Erkrankungen sowie Erkrankungen der Atemwege oder der Augen.

10. Kosmetische Zusammensetzung für die Körper- und Haarpflege, dadurch gekennzeichnet, daß sie in einem geeigneten kosmetischen Träger mindestens eine Verbindung nach einem der Ansprüche 1 bis 4 enthält.

11. Zusammensetzung nach Anspruch 10, dadurch gekennzeichnet, daß sie, bezogen auf das Gesamtgewicht der Zusammensetzung, 0,0001 bis etwa 3 Gew.-% mindestens einer Verbindung nach einem der Ansprüche 1 bis 4 enthält.

## Patentansprüche (Patentansprüche für folgende(n) Vertragsstaat(en): GR)

1. Stereospezifisches Retinoid-Derivat der Formel 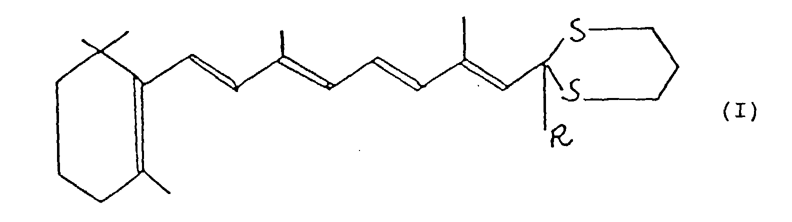 in welcher R für H oder einen C₁-C₄-Thioalkyl-Radikal steht.

2. All-trans-Retinoid-Derivat nach Anspruch 1 der Formel

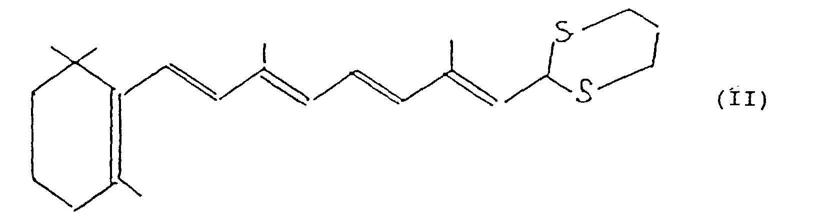

3. 13-cis-Retinoid-Derivat nach Anspruch 1 der Formel

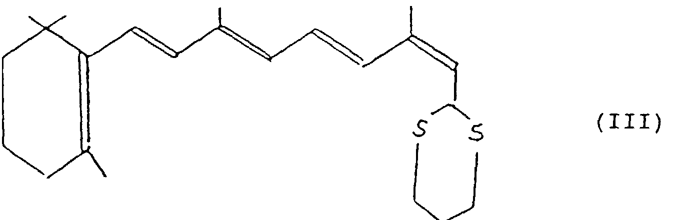

4. All-trans-Retinoid-Derivat nach Anspruch 1 der Formel

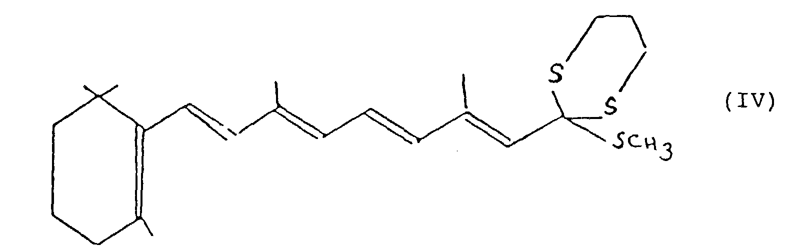

5. Verfahren zur Herstellung eines stereospezifischen Retinoids nach einem der Ansprüche 1 bis 4, dadurch gekennzeichnet, dass man ein Dithianpropenal der Formel 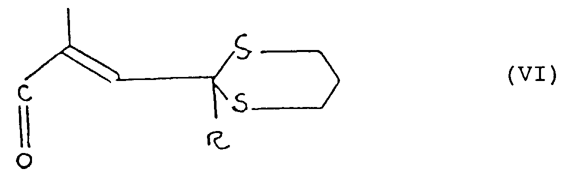 in welcher R dieselbe Bedeutung wie in Anspruch 1 hat, mit Ethyl-β-ionol der Formel 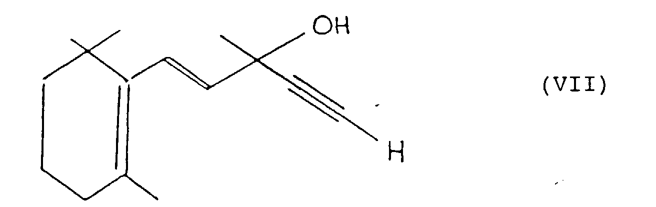 in Anwesenheit eines Organomagnesiums reagieren lässt, so dass man die Acetylenverbindung der Formel 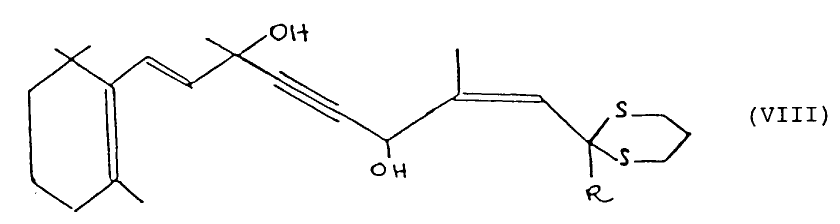 erhält, in der R dieselbe Bedeutung wie oben hat, dann die Acetylenbindung der Verbindung der Formel (VIII) hydrogeniert, um das Diol der Formel 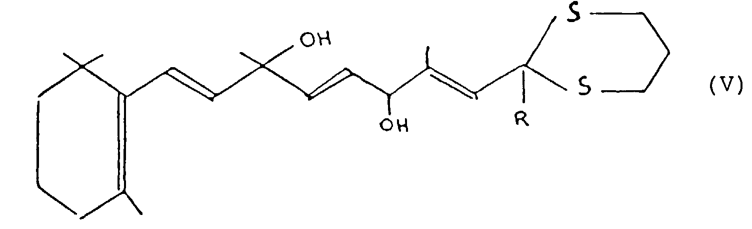 zu erhalten, in der R dieselbe Bedeutung wie in Anspruch 1 hat, und dass man das Diol der Formel (V) mit Hilfe von Titan mit einer "niederen Wertigkeit" reduziert.

6. Verfahren nach Anspruch 5, dadurch gekennzeichnet, dass die Hogdrogenierung der Verbindung der Formel (VIII) mit Hilfe von Lithium- und Aluminiumhydrid durchgeführt wird.

7. Kosmetische Zusammensetzung für die Körper- und Haarpflege, dadurch gekennzeichnet, dass sie in einem geeigneten kosmetischen Träger mindestens eine Verbindung nach einem der Ansprüche 1 bis 4 enthält.

8. Zusammensetzung nach Anspruch 7, dadurch gekennzeichnet, dass sie, bezogen auf das Gesamtgewicht der Zusammensetzung, 0,001 bis etwa 3 Gew.-% mindestens einer Verbindung nach einem der Ansprüche 1 bis 4 enthält.

## Patentansprüche (Patentansprüche für folgende(n) Vertragsstaat(en): ES)

1. Verfahren zur Herstellung eines stereospezifischen Retinoids der Formel: 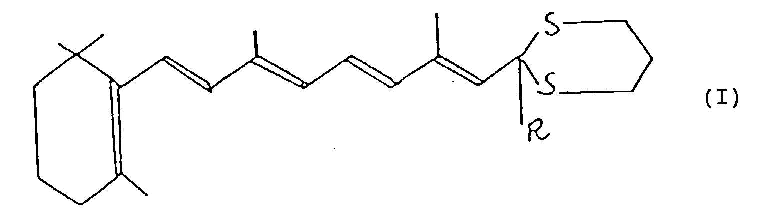 dadurch gekennzeichnet, dass man ein Dithianpropenal der Formel: 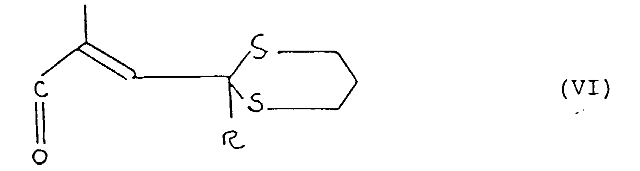 in welcher R für H oder einen C₁-C₄-Thioalkyl-Radikal steht mit Ethyl-β-ionol der Formel 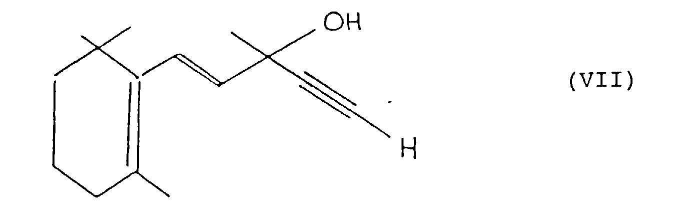 in Anwesenheit eines Organomagnesiums reagieren lässt, so dass man die Acetylenverbindung der Formel: 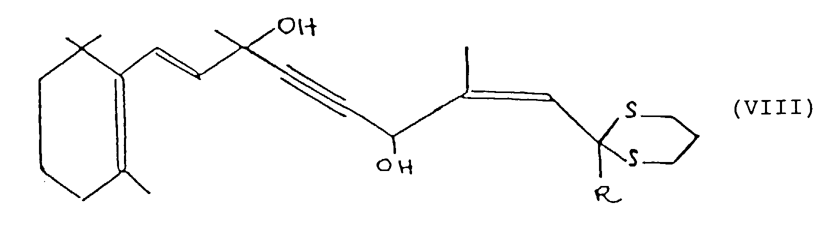 erhält, in der R dieselbe Bedeutung wie oben hat, und dass man das Diol der Formel (V) mit Hilfe von Titan mit einer "niederen Wertigkeit" reduziert.

2. Verfahren nach Anspruch 2 dadurch gekennzeichnet, dass die Hydrogenierung der Verbindung der Formel (VIII) mit Hilfe von Lithium- und Aluminiumhydrid durchgeführt wird.

3. Kosmetische Zusammernstzung für die Körper- und Haarpflege, dadurch gekennzeichnet, dass sie in einem geeigneten kosmetischen Träger mindestens eine durch des Verfahren nach einem der Ansprüche 1 oder 2 hergestellte Verbindung enthält.

4. Kosmetische Zusammensetzung nach Anspruch 3, dadurch gekennzeichnet, dass sie, bezogen auf das Gesamtgewicht der Zusammensetzung, 0,0001 bis etwa 3 Gew.-% mindenstens eine durch des Verfahren nach einem der Ansprüche 1 oder 2 hergestellte Verbindung enthält.

## Claims (Claims for the following Contracting State(s): AT, BE, CH, DE, DK, FR, GB, IT, LI, NL, PT, SE)

1. Stereospecific retinoic derivative of formula: 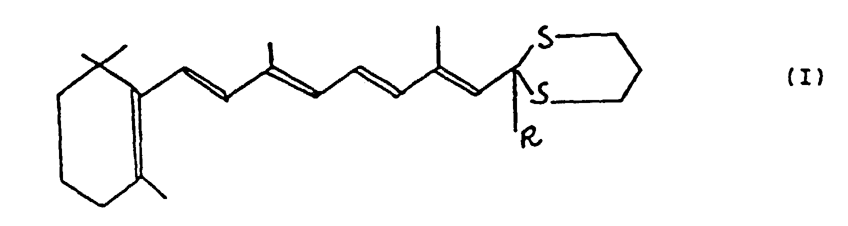 in which formula R is H or a C₁-C₄ thioalkyl radical.

2. All-trans retinoic derivative according to Claim 1, of formula:

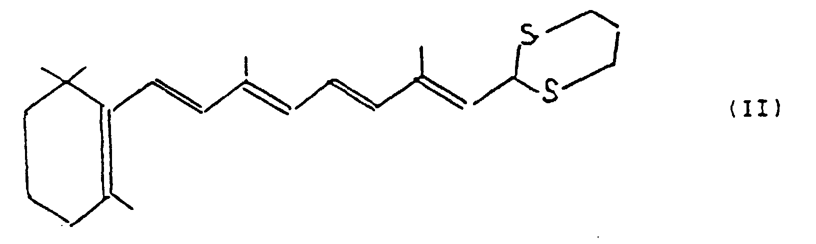

3. 13-cis retinoic derivative according to Claim 1, of formula:

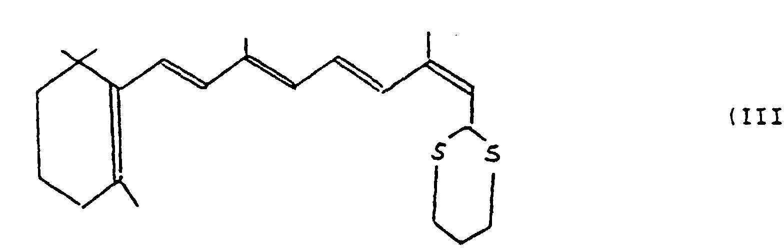

4. All-trans retinoic derivative according to Claim 1, of formula:

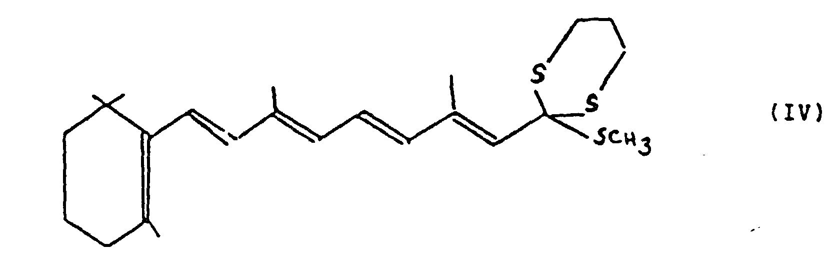

5. Process for preparing a stereospecific retinoid according to one of Claims 1 to 4, characterized in that a dithianepropenal of formula: 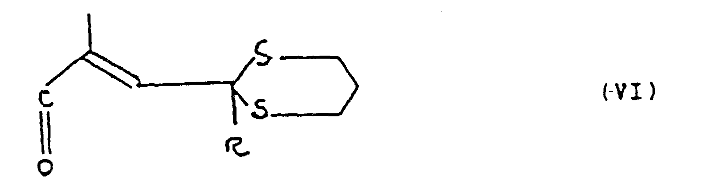 where R has the same meaning as in Claim 1, is reacted with ethyl-β-ionol of formula: 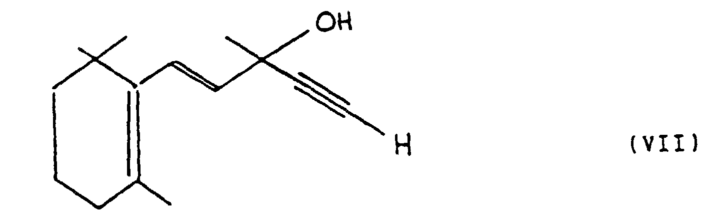 in the presence of an organomagnesium compound so as to obtain the acetylenic compound of formula: 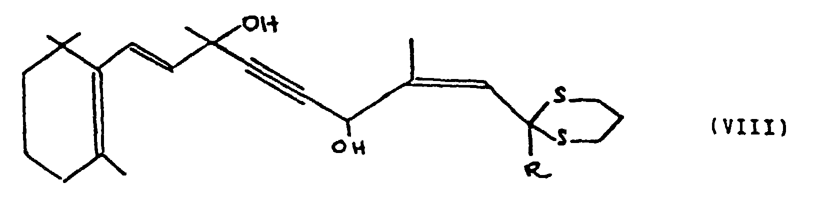 where R has the same meaning as above, and the acetylenic bond of the compound of formula (VIII) is then hydrogenated to obtain the diol of formula: 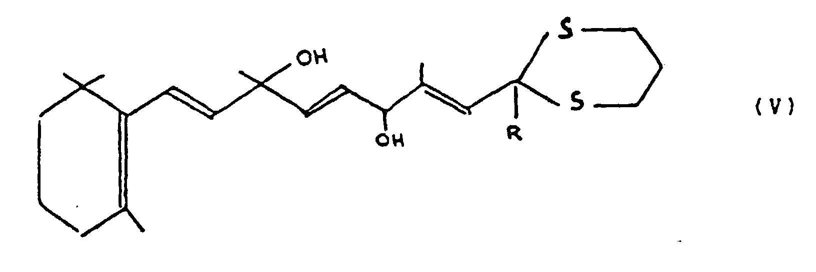 where R has the same meaning as in Claim 1, and in that the diol of formula (V) is reduced using titanium having a "low valency".

6. Process according to Claim 5, characterized in that the hydrogenation of the compound of formula (VIII) is performed using lithium aluminium hydride.

7. Pharmaceutical composition, characterized in that it contains, in a vehicle suitable for enteral, parenteral, topical or ocular administration, at least one compound according to one of Claims 1 to 4.

8. Composition according to Claim 7, characterized in that it contains from 0.0001 to approximately 5% by weight, relative to the total weight of the composition, of at least one compound according to one of Claims 1 to 4.

9. Use of a pharmaceutical composition according to either of Claims 7 and 8 for obtaining a medicinal product for the treatment of dermatological, rheumatic, respiratory or ophthalmological complaints.

10. Cosmetic composition for body and hair hygiene, characterized in that it contains, in a suitable cosmetic vehicle, at least one compound according to one of Claims 1 to 4.

11. Composition according to Claim 10, characterized in that it contains 0.0001 to approximately 3% by weight, relative to the total weight of the composition, of at least one compound according to one of Claims 1 to 4.

## Claims (Claims for the following Contracting State(s): GR)

1. Stereospecific retinoic derivative of formula: 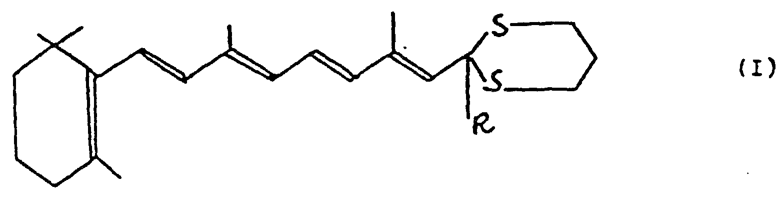 in which formula R is H or a C₁-C₄ thioalkyl radical.

2. All-trans retinoic derivative according to Claim 1, of formula:

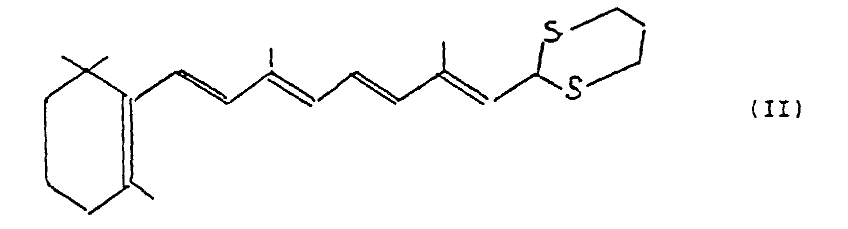

3. 13-cis retinoic derivative according to Claim 1, of formula:

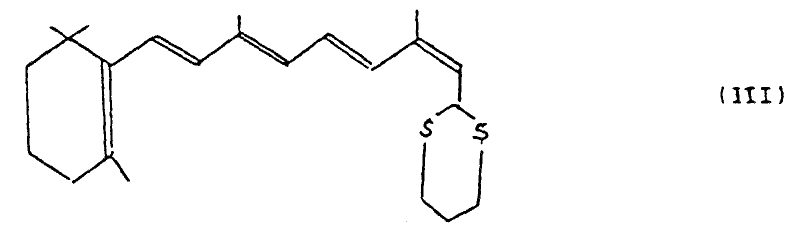

4. All-trans retinoic derivative according to Claim 1, of formula:

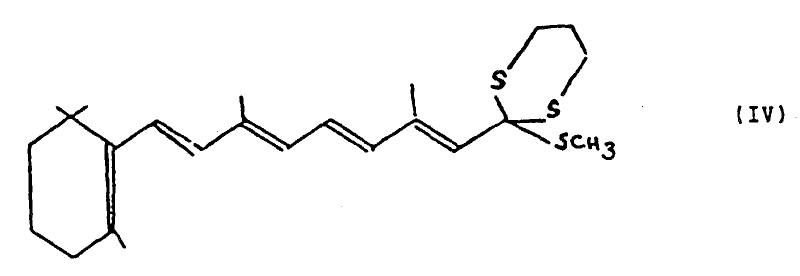

5. Process for preparing a stereospecific retinoid according to one of Claims 1 to 4, characterized in that a dithianepropenal of formula: 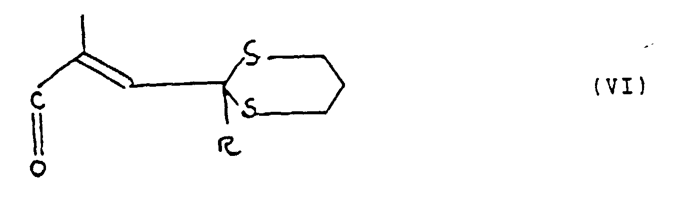 where R has the same meaning as in Claim 1, is reacted with ethyl-β-ionol of formula: 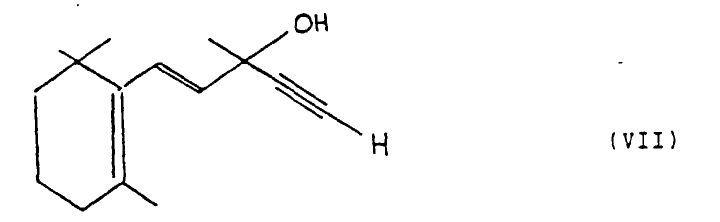 in the presence of an organomagnesium compound so as to obtain the acetylenic compound of formula: 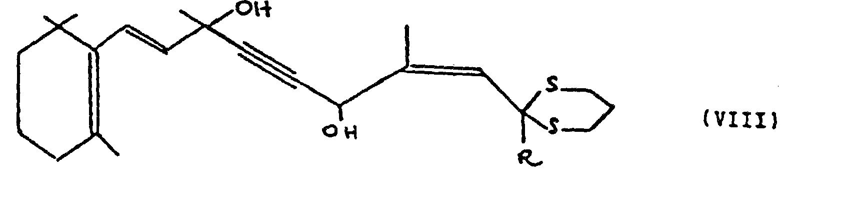 where R has the same meaning as above, and the acetylenic bond of the compound of formula (VIII) is then hydrogenated to obtain the diol of formula: 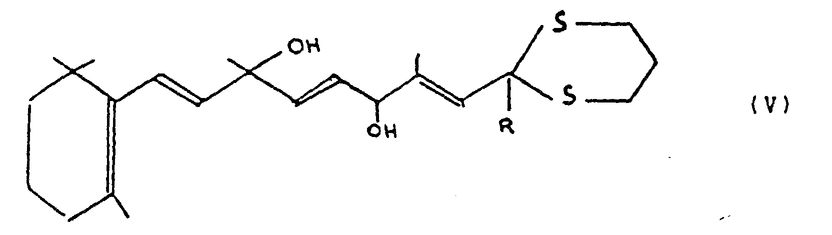 where R has the same meaning as in Claim 1, and in that the diol of formula (V) is reduced using titanium having a "low valency".

6. Process according to Claim 5, characterized in that the hydrogenation of the compound of formula (VIII) is performed using lithium aluminium hydride.

7. Cosmetic composition for body and hair hygiene, characterized in that it contains, in a suitable cosmetic vehicle, at least one compound according to one of Claims 1 to 4.

8. Composition according to Claim 7, characterized in that it contains 0.0001 to approximately 3% by weight, relative to the total weight of the composition, of at least one compound according to one of Claims 1 to 4.

## Claims (Claims for the following Contracting State(s): ES)

1. Process for preparing a stereospecific retinoid of formula: 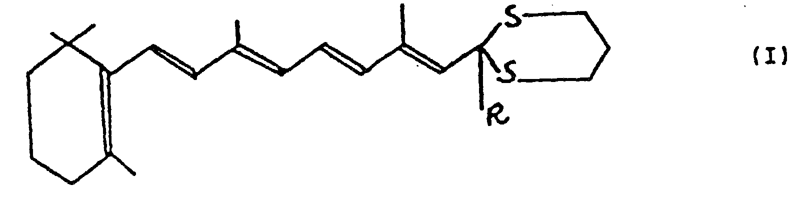 characterized in that a dithianepropenal of formula: 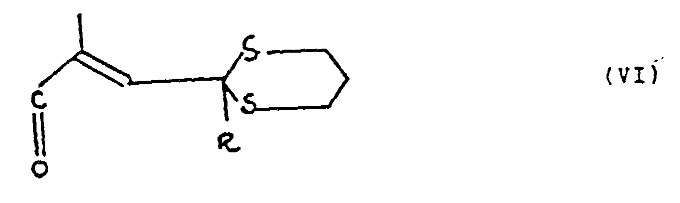 where R is H or a C₁-C₄ thioalkyl radical, is reacted with ethyl-β-ionol of formula: 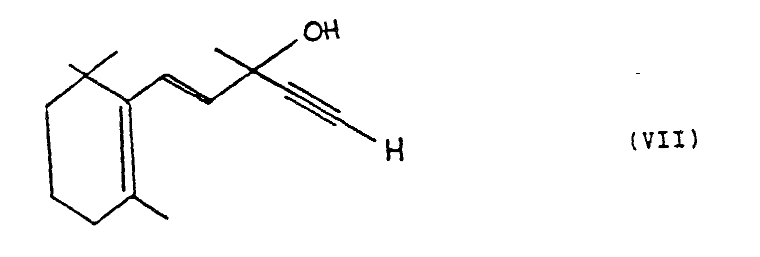 in the presence of an organomagnesium compound, so as to obtain the acetylenic compound of formula: 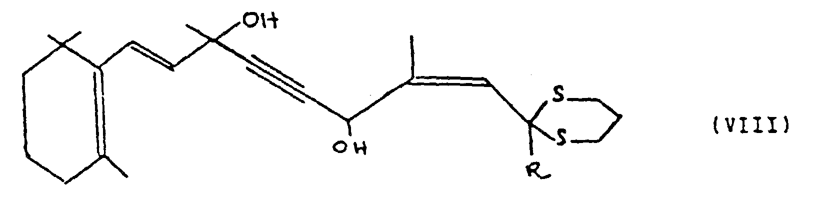 where R has the same meaning as above, and the acetylenic bond of the compound of formula (VIII) is then hydrogenated to obtain the diol of formula: 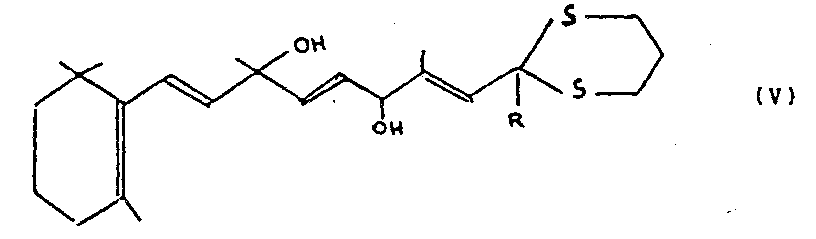 where R is H or a C₁-C₄ thioalkyl radical, and in that the diol of formula (V) is reduced using titanium having a "low valency".

2. Process according to Claim 1, characterized in that the hydrogenation of the compound of formula (VIII) is performed using lithium aluminium hydride.

3. Cosmetic composition for body and hair hygiene, characterized in that it contains, in a suitable cosmetic vehicle, at least one compound obtained by the process of either of Claims 1 and 2.

4. Composition according to Claim 3, characterized in that it contains 0.0001 to approximately 3% by weight, relative to the total weight of the composition, of at least one compound obtained by the process of either of Claims 1 and 2.
